# EUROPÄISCHE PATENTSCHRIFT

(11) **EP 0 763 552 B1**
(45) Veröffentlichungstag und Bekanntmachung des Hinweises auf die Patenterteilung: **19.09.2001**
(21) Anmeldenummer: 96114275.9
(22) Anmeldetag: 06.09.1996
(51) Int. Cl.: C08F 246/00, C08F 220/34, C08F 220/30, C09K 19/38, C07C 69/92

(54) **Vernetzbare, photoaktive Polymermaterialien**
Curable, photoactive polymeric materials
Matériaux polymères durcissables photo-actifs

(30) Priorität: 15.09.1995 CH 261595; 13.03.1996 CH 66496
(43) Veröffentlichungstag der Anmeldung: 19.03.1997
(73) Patentinhaber: Rolic AG, 6301 Zug (CH)
(72) Erfinder: Herr, Rolf-Peter, 79540 Lörrach (DE); Herzog, François, 68120 Richwiller (FR); Schuster, Andreas, 79110 Freiburg (DE)
(74) Vertreter: Groner, Manfred

(56) Entgegenhaltungen:
- EP-A- 0 322 708
- EP-A- 0 611 786
- DE-A- 4 408 170

## Beschreibung

Die Erfindung betrifft neue vernetzbare, photoaktive Polymermaterialien mit 3-Aryl-acrylsäureestern und -amiden, sowie deren Verwendung als Orientierungsschichten für Flüssigkristalle und zum Aufbau unstrukturierter bzw. strukturierter optischer Elemente und Mehrschichtsysteme.

Der Orientierungsschicht kommt in (elektro-optischen) Flüssigkristallvorrichtungen eine besondere Bedeutung zu. Sie dient dem Zweck, eine gleichmässige und störungsfreie Ausrichtung der Moleküllängsachsen zu gewährleisten.

Zur Orientierung von Flüssigkristallmolekülen in Flüssigkristallanzeigen (LCD's) verwendet man üblicherweise uniaxial geriebene Polymerorientierungsschichten wie z.B. Polyimid. Die Reibrichtung gibt bei diesem Prozess die Orientierungsrichtung vor. Mit dem Reiben sind jedoch einige gravierende Nachteile verbunden, die die optische Qualität von Flüssigkristallanzeigen stark beeinflussen können. So wird durch das Reiben Staub erzeugt, der zu optischen Fehlstellen im Display führen kann. Gleichzeitig wird die Polymerschicht elektrostatisch aufgeladen, was beispielsweise bei Thin Film Transistor (TFT)-TN-LCD's die Zerstörung der darunterliegenden Dünnschichttransistoren zur Folge haben kann. Aus diesen Gründen ist die Ausbeute an optisch einwandfreien Displays bei der LCD-Produktion bisher nicht optimal.

Ein weiterer Nachteil des Reibens besteht darin, dass es nicht möglich ist, auf einfache Weise strukturierte Orientierungsschichten herzustellen, da die Orientierungsrichtung beim Reiben nicht lokal variiert werden kann. Durch Reiben können somit hauptsächlich grossflächig einheitlich ausgerichtete Schichten hergestellt werden. Strukturierte Orientierungsschichten sind jedoch in vielen Bereichen der Displaytechnologie und der integrierten Optik von grossem Interesse. Beispielsweise lässt sich damit die Blickwinkelabhängigkeit von Twisted Nematic (TN)-LCD's verbessern.

Seit einiger Zeit sind Orientierungsschichten bekannt, bei denen die Orientierungsrichtung durch Bestrahlung mit polarisiertem Licht vorgegeben werden kann. Dadurch können die dem Reiben inhärenten Probleme umgangen werden. Zusätzlich besteht die Möglichkeit, die Orientierungsrichtung gebietsweise unterschiedlich vorzugeben und damit die Orientierungsschicht zu strukturieren.

Eine Möglichkeit der strukturierten Orientierung von Flüssigkristallen nutzt die Isomerisierungsfähigkeit bestimmter Farbstoffmoleküle aus, um photochemisch durch Einstrahlung mit polarisiertem Licht geeigneter Wellenlänge eine Vorzugsrichtung zu induzieren. Dies wird beispielsweise dadurch erreicht, dass man einem Orientierungspolymer einen Farbstoff zumischt, der dann mit polarisiertem Licht bestrahlt wird. Ein solches Guest/Host-System ist zum Beispiel in US-A-4,974,941 beschrieben. Bei diesem System werden Azobenzole in Polyimidorientierungsschichten eingemischt und anschliessend mit polarisiertem Licht bestrahlt. Flüssigkristalle, die mit der Oberfläche einer so belichteten Schicht in Kontakt sind, werden entsprechend dieser Vorzugsrichtung orientiert. Dieser Orientierungsprozess ist reversibel, d.h. durch nochmaliges Bestrahlen der Schicht mit Licht einer zweiten Polarisationsrichtung lässt sich die bereits eingeschriebene Richtung der Orientierung wieder umdrehen. Da dieser Umorientierungsprozess beliebig oft wiederholt werden kann, sind Orientierungsschichten auf dieser Basis für den Einsatz in LCD's weniger geeignet.

Eine weitere Möglichkeit zur Erzeugung hochaufgelöster Orientierungsmuster in flüssigkristallinen Schichten ist in Jpn. J. Appl. Phys. Vol. 31 (1992), 2155 beschrieben. Bei diesem Verfahren wird die durch Bestrahlung mit linear polarisiertem Licht induzierte Dimerisierung polymergebundener photoreaktiver Zimtsäuregruppen zur strukturierten Orientierung von Flüssigkristallen benutzt. Im Gegensatz zu dem oben beschriebenen reversiblen Orientierungsverfahren, wird bei den in Jpn. J. Appl. Phys. Vol. 31 (1992), 2155 beschriebenen photostrukturierbaren Orientierungsschichten ein anisotropes Polymernetzwerk aufgebaut. Diese photoorientierten Polymernetzwerke sind überall dort einsetzbar, wo strukturierte oder unstrukturierte Flüssigkristallorientierungsschichten benötigt werden. Ausser in LCD's kann man solche Orientierungsschichten beipielsweise auch zur Herstellung von sogenannten Hybridschichten verwenden, wie dies in den europäischen Patentanmeldungen EP-A- 0 611 981, EP-A- 0 689 084, EP-A-0689 065 und in der schweizerischen Patentanmeldung No. 2036/95 exemplifiziert wird. Mit diesen Hybridschichten aus photostrukturierten Orientierungspolymeren und vernetzbaren niedermolekularen Flüssigkristallen lassen sich optische Elemente wie etwa nichtabsorptive Farbfilter, Linear- und Zirkularpolarisatoren, optische Verzögerungsschichten, usw. verwirklichen.

Zimtsäurepolymere, die sich prinzipiell zum Aufbau von solchen anisotrop vernetzten, photostrukturierten Orientierungsschichten für Flüssigkristalle eignen, sind beispielsweise in EP-A-611,786 beschrieben. Diese vernetzbaren Zimtsäurederivate sind grundsätzlich über die Carboxylfunktion der Zimtsäure (Phenylacrylsäure) und einen Spacer an die Polymerhauptkette angeknüpft. Die dimerisierbare Acrylestergruppe der Zimtsäure ist in diesen Polymeren immer nach "innen" zum Spacer bzw. Polymerrückgrat hin ausgerichtet, während der aromatische Rest immer vom Polymerrückgrat weg nach "aussen" orientiert ist.

Es zeigt sich nun, dass diese Art der Ausrichtung der Zimtsäure in den bekannten Photopolymeren keineswegs optimal ist. Photochemische Konkurrenzreaktionen wirken sich störend auf die Orientierungsfähigkeit aus. Die bekannten Zimtsäurepolymere zeichnen sich durch eine ungenügende photochemische Langzeitstabilität aus. Beispielsweise führt eine längere UV-Licht Bestrahlung einer vorgefertigten Orientierungsschicht zur Zerstörung der ursprünglich vorhandenen Orientierung. Mehrfachbelichtungen, bei denen eine bereits bestehende Orientierungsschicht mit einem vorgegebenen eingeschriebenen Muster ein weiteres Mal belichtet wird, um die noch unbelichteten Bereiche in eine andere Richtung zu orientieren, können nur durchgeführt werden, wenn die zuvor belichteten Stellen durch eine Maske abgedeckt werden. Ansonsten können die bereits orientierten Bereiche der Schicht ihre Struktur durch photochemische Nebenreaktionen ganz oder teilweise wieder verlieren.

Ein weiterer Nachteil der bisher verwendeten Zimtsäurepolymere besteht darin, dass bei den durch eine einfache Belichtung mit polarisiertem Licht hergestellten Orientierungsoberflächen aus diesen Materialien kein Kippwinkel auftritt. Insbesondere für den Einsatz in LCD's muss aber neben der Orientierungsrichtung auch ein Kippwinkel durch die Orientierungsschicht vermittelt werden.

Bei den oben erwähnten uniaxial geriebenen Polymerorientierungsschichten wird dieser Kippwinkel bereits beim Reibprozess auf der Polymeroberfläche erzeugt. Bringt man einen Flüssigkristall in Kontakt mit einer solchen Oberfläche, so liegen die Flüssigkristallmoleküle nicht parallel sondern geneigt zur Oberfläche, der Kippwinkel wird also auf den Flüssigkristall übertragen. Die Grösse des Kippwinkels wird dabei sowohl durch Reibparameter wie etwa Vorschubgeschwindigkeit und Anpressdruck sowie durch die chemische Struktur des Polymers bestimmt. Für die Herstellung von Flüssigkristallanzeigen sind je nach Typ Kippwinkel zwischen 1° und 15° erforderlich. Die grösseren Kippwinkel werden insbesondere für Supertwisted Nematic (STN) LCD's benötigt, um das Entstehen von sogenannten Fingerprint-Texturen zu vermeiden. In TN- und TFT-TN-LCD's wird durch den Kippwinkel die Dreh- und die Kipprichtung definiert, wodurch "Reverse Twist"- und "Reverse Tilt "- Phänomene verhindert werden. Während Reverse Twist im ungeschalteten Zustand Gebiete mit falschem Drehsinn zur Folge hat, was sich optisch in fleckigem Aussehen der Anzeige bemerkbar macht, macht sich Reverse Tilt vor allem beim Schalten des LCD's durch Verkippen der Flüssigkristalle in unterschiedliche Richtungen optisch sehr störend bemerkbar. Reverse Twist lässt sich durch Dotieren der Flüssigkristallmischung mit einem chiralen Dotierstoff geeigneter Drehrichtung verhindern. Zur Unterdrückung von Reverse Tilt gibt es bisher jedoch keine alternative Möglichkeit zum Einsatz von Orientierungsschichten mit Kippwinkel.

Der Erfindung liegt daher die Aufgabe zugrunde, photoreaktive Polymere herzustellen, die die oben geschilderten Nachteile der bisher verwendeten Zimtsäurepolymere, d.h. die fehlende photochemische Langzeitstabilität und vorallem den fehlenden Tiltwinkel nach Bestrahlung, mit polarisiertem Licht, nicht aufweisen und damit zur Erzeugung stabiler hochaufgelöster Orientierungsmuster befähigt sind.

Überraschenderweise wurde gefunden, dass Seitenkettenpolymere mit 3-Aryl-acrylsäurederivaten als photoreaktiver Einheit, die nicht wie die bisher bekannten Zimtsäurepolymere "innen" über die Carboxylfunktion sondern über den aromatischen Rest an den Spacer bzw. die Polymerhauptkette angebunden sind und deren photoreaktive Acrylateinheiten damit vom Polymerrückgrat weg nach "aussen" orientiert sind, diese Bedingung erfüllen und sich hervorragend als Orientierungsschichten für Flüssigkristalle eignen. Neben einer bedeutend höheren photochemischen Stabilität der Orientierungsschicht und einem Kippwinkel, wird bei den erfindungsgemässen Polymeren auch eine wesentlich bessere Orientierung der Flüssigkristalle erreicht, die z.B. zu einem deutlich verbesserten Kontrast führt.

Gegenstand der vorliegenden Erfindung sind Polymerzusammensetzungen, in denen wiederkehrende Einheiten der allgemeinen Formel I enthalten sind: 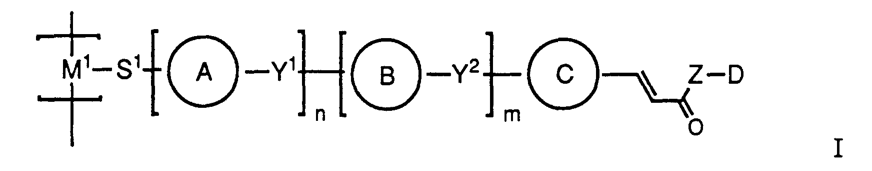 worin
- M¹: eine wiederkehrende Monomereinheit aus der Gruppe: Acrylat, Methacrylat, 2-Chloracrylat, 2-Phenylacrylat; gegebenenfalls durch niederes Alkyl N-substituiertes Acrylamid, Methacrylamid, 2-Chloracrylamid und 2-Phenylacrylamid; Vinylether, Vinylester, Styrol-Derivate, Siloxane;
- S¹: Spacereinheiten, wie beispielsweise eine einfache kovalente Bindung, eine gegebenenfalls einfach oder mehrfach mit Fluor-, Chlor- oder Cyano-substituierte geradkettige oder verzweigte Alkylengruppierung im Folgenden repräsentiert durch -(CH₂)ᵣ-, sowie -(CH₂)ᵣ-O-, -(CH₂)ᵣ-O-(CH₂)ₛ-, -(CH₂)ᵣ-O-(CH₂)ₛ-O-, -(CH₂)ᵣ-CO-, -(CH₂)ᵣ-CO-O-, -(CH₂)ᵣ-O-CO-, -(CH₂)ᵣ-NR²-, -(CH₂)ᵣ-CO-NR²-, -(CH₂)ᵣ-NR²-CO-, -(CH₂)ᵣ-NR²-CO-O-oder -(CH₂)ᵣ-NR²-CO-NR³-, wobei r und s jeweils eine ganze Zahl von 1 bis 20 ist, mit der Massgabe, dass r + s ≤ 20, und R² und R³ unabhängig voneinander Wasserstoff oder niederes Alkyl;
- Ring A: unsubstituiertes oder gegebenenfalls mit Fluor, Chlor, Cyano, Alkyl oder Alkoxy substituiertes Phenylen, Pyridin-2,5-diyl, Pyrimidin-2,5-diyl, 1,3-Dioxan-2,5-diyl, Cyclohexan-1,4-diyl, Piperidin-1,4-diyl, Piperazin-1,4-diyl;
- Ring B: unsubstituiertes oder gegebenenfalls mit Fluor, Chlor, Cyano, Alkyl oder Alkoxy substituiertes Phenylen, Pyridin-2,5-diyl, Pyrimidin-2,5-diyl, 1,4- bzw. 2,6-Naphthylen, 1,3-Dioxan-2,5-diyl, Cyclohexan-1,4-diyl;
- Y¹,Y²: unabhängig voneinander eine einfache Kovalenzbindung, -(CH₂)ₜ-, -O-, -CO-, -CO-O-, -O-OC-, -NR⁴-, -CO-NR⁴-, -R⁴N-CO-, -(CH₂)ᵤ-O-, -O-(CH₂)ᵤ-, -(CH₂)ᵤ-NR⁴- oder -NR⁴-(CH₂)ᵤ-, worin
- R⁴: Wasserstoff oder niederes Alkyl;
- t: eine ganze Zahl von 1 bis 4;
- u: eine ganze Zahl von 1 bis 3;
- m, n: unabhängig voneinander 0 oder 1;
- Ring C: unsubstituiertes oder gegebenenfalls mit Fluor, Chlor, Cyano, Alkyl oder Alkoxy substituiertes Phenylen, oder Pyrimidin-2,5-diyl, Pyridin-2,5-diyl, 2,5-Thiophenylen, 2,5-Furanylen, 1,4-oder 2,6-Naphthylen;
- Z: -O- oder -NR⁵-, wobei R⁵ Wasserstoff oder niederes Alkyl, oder eine zweite Gruppe der Formel D, wobei
- D: eine gegebenenfalls mit Fluor oder Chlor substituierte geradkettige oder verzweigte Alkylgruppe mit 1 bis 20 Kohlenstoffatomen, ein gegebenenfalls mit Fluor, Chlor, Alkyl oder Alkoxy substituierter Cycloalkylrest mit 3 bis 8 Ringatomen;
bedeuten.

Gegenstand der vorliegenden Erfindung ist auch die Verwendung der erfindungsgemässen Polymere als Orientierungsschicht für Flüssigkristalle, sowie deren Verwendung in optischen Bauelementen, insbesondere zur Herstellung von Hybridschichtelementen.

Die erfindungsgemässen Polymermaterialien sind nur aus wiederkehrenden Einheiten der allgemeinen Formel I aufgebaut (Homopolymere wie in Anspruch 16 definiert oder sie enthalten zusätzlich zu den wiederkehrenden Einheiten der allgemeinen Formel I noch weitere wiederkehrende Einheiten (Copolymere), wie in den Ansprüchen 1,6 und 11 definiert. Die erfindungsgemässen Polymere haben ein Molekulargewicht M_{w} zwischen 1 000 und 5 000 000, vorzugsweise jedoch zwischen 5 000 und 2 000 000, besonders vorteilhaft jedoch zwischen 10 000 und 1 000 000.

Comonomereinheiten für die erfindungsgemässen Polymermaterialien mit C-C-Verknüpfung in der Hauptkette können weitere Strukturen der Formel I und/oder aber auch andere in der Polymerchemie übliche Strukturen wie beispielsweise geradkettige oder verzweigte Alkylester der Acryl- bzw. Methacrylsäure, Allylester der Acryl- bzw. Methacrylsäure, Alkylvinylether bzw. Ester, Phenoxyalkylacrylate bzw. Phenoxyalkylmethacrylate, oder Hydroxyalkylacrylate bzw. Hydroxyalkylmethacrylate, Phenylalkylacrylate bzw. Phenylalkylmethacrylate, wobei die Alkylreste 1 bis 20, vorzugsweise 1 bis 10, insbesondere jedoch 1 bis 6 Kohlenstoffatome haben; Acrylnitril, Methacrylnitril, Styrol, 4-Methylstyrol, und dergleichen sein. Bevorzugte Comonomereinheiten sind Strukturen der Formel I, Alkylester der Acryl- bzw. Methacrylsäure, Hydroxyalkylacrylat, Hydroxyalkylmethacrylat, Acrylnitril, Methacrylnitril oder Styrol, insbesondere jedoch Strukturen der Formel I, Alkylester der Acryl- bzw. Methacrylsäure, Hydroxyalkylacrylat oder Hydroxyalkylmethacrylat.

Comonomereinheiten für Siloxane sind bevorzugt weitere Siloxanstrukturen der Formel I und/oder Dimethylsiloxangruppen.

Der Anteil an Comonomereinheiten in den erfindungsgemässen Polymeren, die nicht einer Struktur der Formel I entsprechen, ist kleiner oder gleich 50 %, vorzugsweise kleiner oder gleich 30 %, insbesondere jedoch kleiner oder gleich 15 %.

Unter dem Ausdruck "Copolymere" werden vorzugsweise statistische Copolymere verstanden, wie beispielsweise Copolymere aus verschiedenen Derivaten der Formel I oder aus Strukturen der Formel I mit Acryl-, Methacrylsäure- oder Styrol-Derivaten. Homopolymere umfassen lineare und cyclische Polymere, wie beispielsweise cyclische Polysiloxane, bevorzugt jedoch lineare Polymere.

### Wiederkehrende Monomereinheiten (M¹) sind beispielsweise

### Acrylate wie

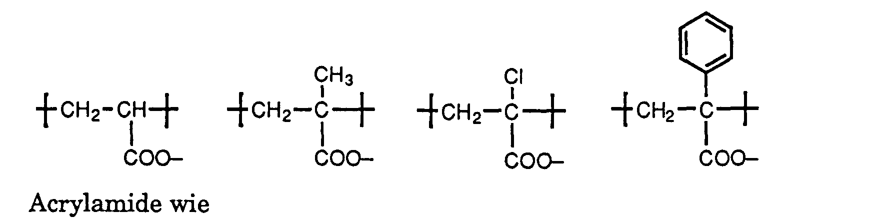

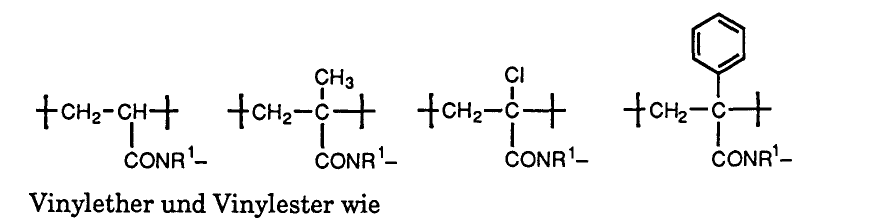

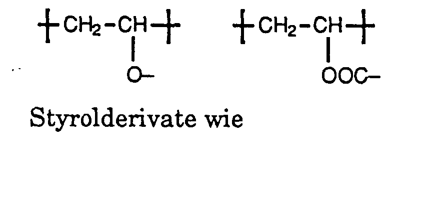

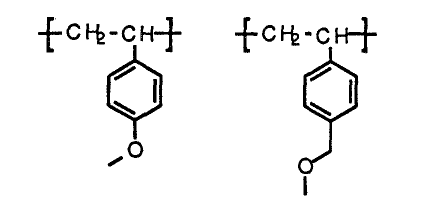

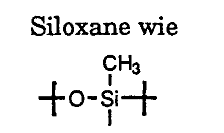

worin R¹ Wasserstoff oder niederes Alkyl bedeutet;

Bevorzugte "Monomereinheiten" M¹ sind Acrylat, Methacrylat, 2-Chloracrylat, Acrylamid, Methacrylamid, 2-Chloracrylamid, Styrol-Derivate und Siloxane.

Besonders bevorzugte "Monomereinheiten" M¹ sind Acrylat, Methacrylat, Styrol-Derivate und Siloxane.

Ganz besonders bevorzugte "Monomereinheiten" M¹ sind Acrylat, Methacrylat und Styrol-Derivate.

Der Ausdruck "niederes Alkyl" für sich alleine genommen oder in Kombination wie "niederes Alkoxy", " Hydroxy- niederes Alkyl", " Phenoxyniederes Alkyl" bezeichnet geradkettige und verzweigte gesättigte Kohlenwasserstoffreste mit 1 bis 6, vorzugsweise mit 1 bis 3 Kohlenstoffatomen, wie Methyl, Ethyl, Propyl oder i-Propyl und dergleichen.

Der Ausdruck "Alkyl" für sich alleine genommen oder in Kombination wie "Alkoxy", bezeichnet geradkettige und verzweigte gesättigte Kohlenwasserstoff-Reste mit bis zu 20 Kohlenstoffatomen.

Bevorzugte "Spacereinheiten" sind im Rahmen der vorliegenden Erfindung eine einfache kovalente Bindung, eine geradkettige oder verzweigte Alkylengruppierung repräsentiert durch -(CH₂)ᵣ-, wobei r eine ganze Zahl von 1 bis 8, insbesondere jedoch 1 bis 6 ist, sowie -(CH₂)ᵣ-O-, -(CH₂)ᵣ-CO-O-, -(CH₂)ᵣ-O-CO-, -(CH₂)ᵣ-NR²-, -(CH₂)ᵣ-CO-NR²- oder -(CH₂)ᵣ-NR²-CO-, worin R² Wasserstoff oder niederes Alkyl bedeutet.

Beispiele von bevorzugten "Spacereinheiten" sind die Einfachbindung, Methylen, 1,2-Ethylen, 1,3-Propylen, 1,4-Butylen, 1,5-Pentylen, 1,6-Hexylen, 1,7-Heptylen, 1,8-Octylen, 1,2-Propylen, 2-Methyl-1,2-propylen, 1,3-Butylen, Ethylenoxy, Ethylenoxycarbonyl, Ethylenoyloxy, Proylenoxy, Propylenoxycarbonyl, Propylenoyloxy, Butylenoxy, Butylenoxycarbonyl, Butylenoyloxy, Pentylenoxy, Pentylenoxycarbonyl, Pentylenoyloxy, Hexylenoxy, Hexylenoxycarbonyl, Hexylenoyloxy, Heptylenoxy, Heptylenoxycarbonyl, Heptylenoyloxy, Octylenoxy, Octylenoxycarbonyl, Octylenoyloxy, Ethylenamino, Propylenamino, Butylenamino, Pentylenamino, Hexylenamino, Heptylenamino, Octylenamino, Ethylenaminocarbonyl, Propylenaminocarbonyl, Butylenaminocarbonyl, Pentylenaminocarbonyl, Hexylenaminocarbonyl, Heptylenaminocarbonyl, Octylenaminocarbonyl, Ethylencarbonylamino, Propylencarbonylamino, Butylencarbonylamino, Pentylencarbonylamino, Hexylencarbonylamino, Heptylencarbonylamino, Octylencarbonylamino und dergleichen.

Besonders bevorzugte "Spacereinheiten" sind eine einfache kovalente Bindung, eine geradkettige Alkylengruppierung repräsentiert durch -(CH₂)ᵣ-, wobei r eine ganze Zahl von 1 bis 6 ist, sowie -(CH₂)ᵣ-O-, -(CH₂)ᵣ-CO-O- und -(CH₂)ᵣ-O-CO-.

Beispiele besonders bevorzugter "Spacereinheiten" sind die Einfachbindung, Methylen, 1,2-Ethylen, 1,3-Propylen, 1,4-Butylen, 1,5-Pentylen, 1,6-Hexylen, Ethylenoxy, Ethylenoxycarbonyl, Ethylenoyloxy, Proylenoxy, Propylenoxycarbonyl, Propylenoyloxy, Butylenoxy, Butylenoxycarbonyl, Butylenoyloxy, Pentylenoxy, Pentylenoxycarbonyl, Pentylenoyloxy, Hexylenoxy, Hexylenoxycarbonyl und Hexylenoyloxy.

Der Ausdruck "unsubstituiertes oder gegebenenfalls mit Fluor, Chlor, Cyano, Alkyl oder Alkoxy substituiertes Phenylen" umfasst im Rahmen der der vorliegenden Erfindung unsubstituiertes bzw. mit Fluor, Chlor, Cyano, Alkyl oder Alkoxy, vorzugsweise mit Fluor, Methyl, Ethyl, Propyl, Butyl, Methoxy, Ethoxy, Propoxy, Butoxy oder Cyano einfach oder mehrfach substituiertes 1,2-, 1,3- oder 1,4-Phenylen. Bevorzugt ist 1,3- oder 1,4-Phenylen, insbesondere jedoch 1,4-Phenylen.

Beispiele bevorzugter Phenylenreste sind 1,3-, bzw. 1,4-Phenylen, 4- bzw. 5-Methyl-1,3-phenylen, 4- bzw. 5-Methoxy-1,3-phenylen, 4- bzw. 5-Ethyl-1,3-phenylen, 4- bzw. 5-Ethoxy-1,3-phenylen, 2- bzw. 3-Methyl-1,4-phenylen, 2-bzw. 3-Ethyl-1,4-phenylen, 2- bzw. 3-Propyl-1,4-phenylen, 2- bzw. 3-Butyl-1,4-phenylen, 2- bzw. 3-Methoxy-1,4-phenylen, 2- bzw. 3-Ethoxy-1,4-phenylen, 2-bzw. 3-Propoxy-1,4-phenylen, 2- bzw. 3-Butoxy-1,4-phenylen, 2,3-, 2,6- bzw. 3,5-Dimethyl-1,4-phenylen, 2,6- bzw. 3,5-Dimethoxy-1,4-phenylen, 2- bzw. 3-Fluor-1,4-phenylen, 2,3-, 2,6- bzw. 3,5-Difluor-1,4-phenylen, 2- bzw. 3-Chlor-1,4-phenylen, 2,3-, 2,6- bzw. 3,5-Dichlor-1,4-phenylen, 2- bzw. 3-Cyano-1,4-phenylen und dergleichen.

Erfindungsgemässe Polymerzusammensetzungen sind Copolymerzusammensetzungen mit wiederkehrenden Einheiten der allgemeinen Formel Ia, 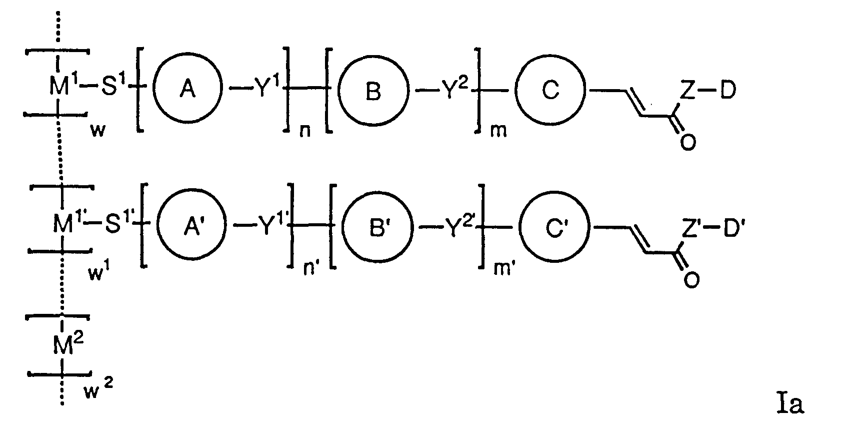 worin
- M¹, M^{1'}: unabhängig voneinander eine wiederkehrende Monomereinheit aus der Gruppe Acrylat, Methacrylat, 2-Chloracrylat, 2-Phenylacrylat, gegebenenfalls durch niederes Alkyl N-substituiertes Acrylamid, Methacrylamid, 2-Chloracrylamid und 2-Phenylacrylamid; Vinylether, Vinylester, Styrol-Derivate, Siloxane;
- S¹, S^{1'}: unabhängig voneinander Spacereinheiten, wie beispielsweise eine einfache kovalente Bindung, eine gegebenenfalls einfach oder mehrfach mit Fluor-, Chlor- oder Cyano-substituierte geradkettige oder verzweigte Alkylengruppierung im Folgenden repräsentiert durch -(CH₂)ᵣ-, sowie -(CH₂)ᵣ-O-, -(CH₂)ᵣO-(CH₂)ₛ-, -(CH₂)ᵣ-O-(CH₂)ₛ-O-, -((CH₂)ₛCO-, -(CH₂)ᵣ-CO-O-, -(CH₂)ᵣ-O-CO-, -(CH₂)ᵣ-NR²-, -(CH₂)ᵣ-CO-NR²-, -(CH₂)ᵣ-NR²-CO-, -(CH₂)ᵣ-NR²-CO-O- oder -(CH₂)ᵣ-NR²-CO-NR³-, wobei r und s jeweils eine ganze Zahl von 1 bis 20 ist, mit der Massgabe, dass r + s ≤ 20, und R2 und R3 unabhängig voneinander Wasserstoff oder niederes Alkyl;
- Ringe A, A': unabhängig voneinander unsubstituiertes oder gegebenenfalls mit Fluor, Chlor, Cyano, Alkyl oder Alkoxy substituiertes Phenylen, Pyridin-2,5-diyl, Pyrimidin-2,5-diyl, 1,3-Dioxan-2,5-diyl, Cyclohexan-1,4-diyl, Piperidin-1,4-diyl, Piperazin-1,4-diyl;
- Ringe B, B': unabhängig voneinander unsubstituiertes oder gegebenenfalls mit Fluor, Chlor, Cyano, Alkyl oder Alkoxy substituiertes Phenylen, Pyridin-2,5-diyl, Pyrimidin-2,5-diyl, 1,4- bzw. 2,6-Naphthylen, 1,3-Dioxan-2,5-diyl, Cyclohexan-1,4-diyl;
- Y¹,Y²,Y^{1'},Y^{2'}: unabhängig voneinander eine einfache Kovalenzbindung, -(CH₂)ₜ-, -O-, -CO-, -CO-O-, -O-OC-, -NR⁴-, -CO-NR⁴-, -R⁴N-CO-, -(CH₂)ᵤ-O-, -O-(CH₂)ᵤ-, -(CH₂)ᵤ-NR⁴- oder -NR⁴-(CH₂)ᵤ-, worin
- R⁴, R^{4'}: unabhängig voneinander Wasserstoff oder niederes Alkyl;
- t, t': unabhängig voneinander eine ganze Zahl von 1 bis 4;
- u, u: unabhängig voneinander eine ganze Zahl von 1 bis 3;
- m, m', n, n': unabhängig voneinander 0 oder 1;
- Ringe C, C': unabhängig voneinander unsubstituiertes oder gegebenenfalls mit Fluor, Chlor, Cyano, Alkyl oder Alkoxy substituiertes Phenylen, oder Pyrimidin-2,5-diyl, Pyridin-2,5-diyl, 2,5-Thiophenylen, 2,5-Furanylen, 1,4- oder 2,6-Naphthylen;
- Z, Z': unabhängig voneinander -O- oder -NR⁵-, wobei R⁵ Wasserstoff oder niederes Alkyl, oder eine zweite Gruppe der Formel D, wobei
- D, D': eine gegebenenfalls mit Fluor oder Chlor substituierte geradkettige oder verzweigte Alkylengruppe mit 1 bis 20 Kohlenstoffatomen, ein gegebenenfalls mit Fluor, Chlor, Alkyl oder Alkoxy substituierter Cycloalkylrest mit 3 bis 8 Ringatomen;
- M²: eine wiederkehrende Monomereinheit aus der Gruppe Acrylat, Methacrylat, 2-Chloracrylat, 2-Phenylacrylat, gegebenenfalls durch niederes Alkyl N-substituiertes Acrylamid, Methacrylamid, 2-Chloracrylamid und 2-Phenylacrylamid; Vinylether, Vinylester; geradkettige oder verzweigte Alkylester der Acryl- bzw. Methacrylsäure, Allylester der Acryl- bzw. Methacrylsäure, Alkylvinylether bzw. Ester, Phenoxyalkylacrylate bzw. Phenoxyalkylmethacrylate oder Hydroxyalkylacrylate bzw. Hydroxyalkylmethacrylate Phenylalkylacrylate bzw. Phenylalkylmethacrylate, wobei die Alkylreste 1 bis 20, vorzugsweise 1 bis 10, insbesondere jedoch 1 bis 6 Kohlenstoffatome haben; Acrylnitril, Methacrylnitril, Styrol, 4-Methylstyrol oder Siloxane bedeuten; und
w, w¹ und w² Molenbrüche der Comonomeren mit 0< w <1, 0 <w¹<1 und 0<w²≤ 0,5 sind.

Bevorzugt sind Copolymerzusammensetzungen mit wiederkehrenden Einheiten der allgemeinen Formel Ia, worin
- M¹, S¹, M^{1'}, S^{1'} und M²: die oben angegebenen Bedeutungen haben; und
- Ringe A, A': unabhängig voneinander unsubstituiertes oder gegebenenfalls mit Fluor, Chlor, Cyano, Alkyl oder Alkoxy substituiertes Phenylen, Pyridin-2,5-diyl, Pyrimidin-2,5-diyl oder Cyclohexan-1,4-diyl;
- Ringe B, B': unabhängig voneinander unsubstituiertes oder gegebenenfalls mit Fluor, Chlor, Cyano, Alkyl oder Alkoxy substituiertes Phenylen, Pyridin-2,5-diyl, Pyrimidin-2,5-diyl, 1,4- bzw. 2,6-Naphthylen oder Cyclohexan-1,4-diyl;
- Y¹, Y², Y^{1'}, Y^{2'}: unabhängig voneinander eine einfache Kovalenzbindung, -CH₂CH₂-, -O-, -CH₂-O-, -O-CH₂-, -CO-O- oder -O-OC-;
- m, n, m', n': unabhängig voneinander 0 oder 1;
- Ringe C, C': unsubstituiertes oder gegebenenfalls mit Fluor, Chlor, Cyano, Alkyl oder Alkoxy substituiertes Phenylen, oder Pyrimidin-2,5-diyl, Pyridin-2,5-diyl, 2,5-Furanylen oder 1,4-oder 2,6-Naphthylen;
- Z, Z': -O-;
- D, D': eine geradkettige oder verzweigte Alkylengruppe mit 1 bis 20, insbesondere mit 1 bis 12 Kohlenstoffatomen oder ein gegebenenfalls mit Alkyl oder Alkoxy, insbesondere mit Methyl oder Methoxy, substituierter Cycloalkylrest mit 5 oder 6 Ringatomen bedeuten; und
- w, w¹ und w²: Molenbrüche der Comonomeren mit 0< w <1, 0 <w¹<1 und 0<w²≤0,5 sind.

Besonders bevorzugt sind Copolymerzusammensetzungen mit wiederkehrenden Einheiten der allgemeinen Formel Ia, worin n und n' = 0 ist und

M¹ und S¹ sowie M^{1'} und S^{1'} und M² die oben angegebenen Bedeutungen haben; und
- Ringe B, B': unabhängig voneinander unsubstituiertes oder gegebenenfalls mit Fluor, Chlor, Cyano, Alkyl oder Alkoxy substituiertes Phenylen, Pyridin-2,5-diyl, Pyrimidin-2,5-diyl oder Cyclohexan-1,4-diyl;
- Y²,Y^{2'}: unabhängig voneinander eine einfache Kovalenzbindung, -CO-O- oder -O-OC-;
- m, m': 0 oder 1;
- Ringe C, C': unabhängig voneinander unsubstituiertes oder gegebenenfalls mit Fluor, Chlor, Cyano, Alkyl oder Alkoxy substituiertes Phenylen oder 1,4- oder 2,6-Naphthylen;
- n, n': 0;
- Z, Z': -O-; und
- D, D': unabhängig voneinander eine geradkettige oder verzweigte Alkylengruppe mit 1 bis 12 Kohlenstoffatomen bedeuten; und
- w, w¹ und w²: Molenbrüche der Comonomeren mit 0< w <1, 0 <w¹<1 und 0<w²≤ 0,5 sind.

Eine Copolymerzusammensetzung der Formel Ia lässt sich beispielsweise analog Beispiel 3 herstellen, nämlich

Poly [1-[2-[4-[(E)-2-methoxycarbonyl-vinyl]-phenoxy]-ethoxycarbonyl]-1-methyl-ethylen-co-1-[2-[4-[(E)-2-propoxycarbonyl-vinyl]-phenoxy]-ethoxycarbonyl]-1-methyl-ethylen-co-1-[2-hydroxy-ethoxycarbonyl]-1-methyl-ethylen] (Beispiel 3).

Weitere erfindungsgemässe Copolymerzusammensetzungen der allgemeinen Formel I mit in der Polymerchemie üblichen Strukturen bestehen aus Verbindungen der Formel Ib, 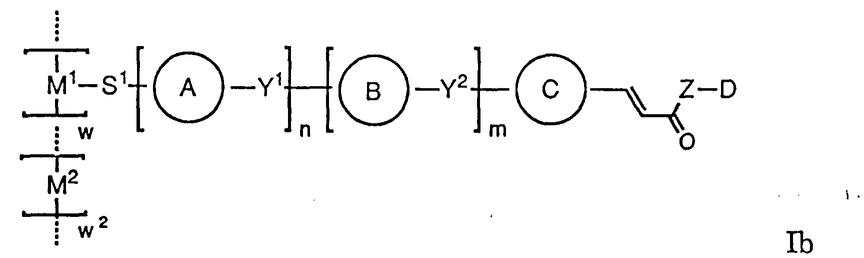 worin

M¹, M², S¹, A, B, C, D, Z, Y¹, Y², m und n die oben genannten Bedeutungen haben; und
w und w ² Molenbrüche der Comonomeren mit 0 < w < 1 und 0 < w ²≤0,5 sind.

Bevorzugt sind Copolymerzusammensetzungen mit wiederkehrenden Einheiten der allgemeinen Formel Ib, worin
- M¹, M² und S¹: die oben angegebenen Bedeutungen haben; und
- Ring A: unsubstituiertes oder gegebenenfalls mit Fluor, Chlor, Cyano, Alkyl oder Alkoxy substituiertes Phenylen, Pyridin-2,5-diyl, Pyrimidin-2,5-diyl oder Cyclohexan-1,4-diyl;
- Ring B: unsubstituiertes oder gegebenenfalls mit Fluor, Chlor, Cyano, Alkyl oder Alkoxy substituiertes Phenylen, Pyridin-2,5-diyl, Pyrimidin-2,5-diyl, 1,4- bzw. 2,6-Naphthylen oder Cyclohexan-1,4-diyl;
- Y¹, Y²: unabhängig voneinander eine einfache Kovalenzbindung, -CH₂CH₂-, -O-, -CH₂-O-, -O-CH₂-, -CO-O- oder -O-OC-;
- m, n: unabhängig voneinander 0 oder 1;
- Ring C: unsubstituiertes oder gegebenenfalls mit Fluor, Chlor, Cyano, Alkyl oder Alkoxy substituiertes Phenylen, oder Pyrimidin-2,5-diyl, Pyridin-2,5-diyl, 2,5-Furanylen oder 1,4- oder 2,6-Naphthylen;
- Z: -O-; und
- D: eine geradkettige oder verzweigte Alkylengruppe mit 1 bis 20, insbesondere mit 1 bis 12 Kohlenstoffatomen oder ein gegebenenfalls mit Alkyl oder Alkoxy, insbesondere mit Methyl oder Methoxy, substituierter Cycloalkylrest mit 5 oder 6 Ringatomen bedeuten; und
- w und w²: Molenbrüche der Comonomeren mit 0 < w < 1 und 0 < w²≤ 0,5 sind.

Besonders bevorzugt sind Copolymerzusammensetzungen mit wiederkehrenden Einheiten der allgemeinen Formel Ib, worin n = 0 ist;
- M¹, M² und S¹: die oben angegebenen Bedeutungen haben;
- Ring B: unsubstituiertes oder gegebenenfalls mit Fluor, Chlor, Cyano, Alkyl oder Alkoxy substituiertes Phenylen, Pyridin-2,5-diyl, Pyrimidin-2,5-diyl oder Cyclohexan-1,4-diyl;
- Y²: eine einfache Kovalenzbindung, -CO-O- oder -O-OC-;
- m: 0 oder 1;
- Ring C: unsubstituiertes oder gegebenenfalls mit Fluor, Chlor, Cyano, Alkyl oder Alkoxy substituiertes Phenylen oder 1,4- oder 2,6-Naphthylen;
- n: 0;
- Z: -O-; und
- D: eine geradkettige oder verzweigte Alkylengruppe mit 1 bis 12 Kohlenstoffatomen bedeuten; und
- w und w²: Molenbrüche der Comonomeren mit 0 < w < 1 und 0 < w²≤ 0,5 sind.

Solche besonders bevorzugten Copolymerzusammensetzungen mit in der Polymerchemie üblichen Strukturen der Formel Ib werden in Beispiel 7 beschrieben, nämlich
Poly [1-[6-[4-[4-[(E)-2-methoxycarbonyl-vinyl]-phenoxycarbonyl]-phenoxy]-hexyloxycarbonyl]-1-methyl-ethylen-co-1-[2-ethylhexyloxycarbonyl]-1-methyl-ethylen] (Beispiel 7);
Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-ethoxycarbonyl-1-methyl-ethylen] (Beispiel 7);
Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-[2-ethylhexyloxycarbonyl-1-methyl-ethylen] (Beispiel 7).

Weitere erfindungsgemässe Copolymerzusammensetzungen der allgemeinen Formel I bestehen aus wiederkehrenden Einheiten der allgemeinen Formel Ic, 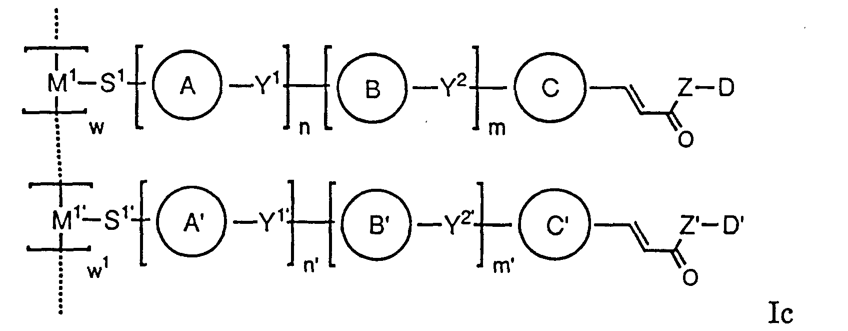 worin

M¹, S¹, A, B, C, D, Z, Y¹, Y², m und n sowie M^{1'}, S^{1'}, A', B', C', D', Z', Y^{1'}, Y^{2'}, m' und n' die oben angegebenen Bedeutungen haben; und
w und w¹ Molenbrüche der Comonomeren mit 0< w <1, 0 <w¹<1 sind.

Bevorzugt sind Copolymerzusammensetzungen mit wiederkehrenden Einheiten der allgemeinen Formel Ic, worin
- M¹ und S¹ sowie M^{1'} und S^{1'}: die oben angegebenen Bedeutungen haben; und
- Ringe A, A': unabhängig voneinander unsubstituiertes oder gegebenenfalls mit Fluor, Chlor, Cyano, Alkyl oder Alkoxy substituiertes Phenylen, Pyridin-2,5-diyl, Pyrimidin-2,5-diyl oder Cyclohexan-1,4-diyl;
- Ringe B, B': unabhängig voneinander unsubstituiertes oder gegebenenfalls mit Fluor, Chlor, Cyano, Alkyl oder Alkoxy substituiertes Phenylen, Pyridin-2,5-diyl, Pyrimidin-2,5-diyl, 1,4- bzw. 2,6-Naphthylen oder Cyclohexan-1,4-diyl;
- Y¹,Y²,Y^{1'},Y^{2'}: unabhängig voneinander eine einfache Kovalenzbindung, -CH₂CH₂-, -O-, -CH₂-O-, -O-CH₂-, -CO-O- oder -O-OC-;
- m, n, m', n': unabhängig voneinander 0 oder 1;
- Ringe C, C': ' unabhängig voneinander unsubstituiertes oder gegebenenfalls mit Fluor, Chlor, Cyano, Alkyl oder Alkoxy substituiertes Phenylen, oder Pyrimidin-2,5-diyl, Pyridin-2,5-diyl, 2,5-Furanylen oder 1,4- oder 2,6-Naphthylen;
- Z, Z': -O-;
- D, D': unabhängig voneinander eine geradkettige oder verzweigte Alkylengruppe mit 1 bis 20, insbesondere mit 1 bis 12 Kohlenstoffatomen oder ein gegebenenfalls mit Alkyl oder Alkoxy, insbesondere mit Methyl oder Methoxy, substituierter Cycloalkylrest mit 5 oder 6 Ringatomen bedeuten; und
- w und w¹: Molenbrüche der Comonomeren mit 0< w <1, 0 <w¹<1 sind.

Besonders bevorzugt sind Copolymerzusammensetzungen mit wiederkehrenden Einheiten der allgemeinen Formel Ic, worin n und n' = 0 ist und
- M¹ und S¹ sowie M^{1'} und S^{1'}: die oben angegebenen Bedeutungen haben; und
- Ringe B, B': unabhängig voneinander unsubstituiertes oder gegebenenfalls mit Fluor, Chlor, Cyano, Alkyl oder Alkoxy substituiertes Phenylen, Pyridin-2,5-diyl, Pyrimidin-2,5-diyl oder Cyclohexan-1,4-diyl;
- Y²,Y^{2'}: unabhängig voneinander eine einfache Kovalenzbindung, -CO-O- oder -O-OC-;
- m, m': unabhängig voneinander 0 oder 1;
- Ringe C, C': unabhängig voneinander unsubstituiertes oder gegebenenfalls mit Fluor, Chlor, Cyano, Alkyl oder Alkoxy substituiertes Phenylen oder 1,4- oder 2,6-Naphthylen;
- n, n': 0;
- Z, Z': -O-; und
- D, D': unabhängig voneinander eine geradkettige oder verzweigte Alkylgruppe mit 1 bis 12 Kohlenstoffatomen bedeuten; und
- w und w¹: Molenbrüche der Comonomeren mit 0< w <1, 0 <w¹<1 sind.

Eine solche besonders bevorzugte Copolymerzusammensetzung wird in Beispiel 9 beschrieben, nämlich

Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-[2-[4-[2-methoxy-4-[(E)-2-methoxycarbonyl-vinyl]-phenoxycarbonyl]-phenoxy]-ethoxycarbonyl]-1-methyl-ethylen] (Beispiel 9).

Erfindungsgemässe Homopolymerzusammensetzungen sind Homopolymerzusammensetzungen mit wiederkehrenden Einheiten der allgemeinen Formel I, 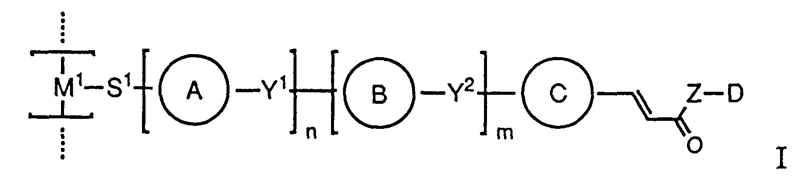 worin

M¹, S¹, A, B, C, D, Z, Y¹, Y², m und n die oben angegebenen Bedeutungen haben.

Eine solche bevorzugte Homopolymerzusammensetzung ist beispielsweise:

Poly [1-[3-[4-[4'-[(E)-2-methoxycarbonyl-vinyl]-biphenyl-4-yl]-cyclohexyl]-propoxycarbonyl]-1-methyl-ethylen] ( Beispiel 6).

Besonders bevorzugt sind Homopolymerzusammensetzungen mit wiederkehrenden Einheiten der allgemeinen Formel I, worin
- M¹ und S¹: die in oben angegebenen Bedeutungen haben; und
- Ring A: unsubstituiertes oder gegebenenfalls mit Fluor, Chlor, Cyano, Alkyl oder Alkoxy substituiertes Phenylen, Pyridin-2,5-diyl, Pyrimidin-2,5-diyl oder Cyclohexan-1,4-diyl;
- Ring B: unsubstituiertes oder gegebenenfalls mit Fluor, Chlor, Cyano, Alkyl oder Alkoxy substituiertes Phenylen, Pyridin-2,5-diyl, Pyrimidin-2,5-diyl, 1,4- bzw. 2,6-Naphthylen oder Cyclohexan-1,4-diyl;
- Y¹,Y²: unabhängig voneinander eine einfache Kovalenzbindung, -CH₂CH₂-, -O-, -CH₂-O-, -O-CH₂-, -CO-O- oder -O-OC-;
- m, n: unabhängig voneinander 0 oder 1;
- Ring C: unsubstituiertes oder gegebenenfalls mit Fluor, Chlor, Cyano, Alkyl oder Alkoxy substituiertes Phenylen, oder Pyrimidin-2,5-diyl, Pyridin-2,5-diyl, 2,5-Furanylen oder 1,4- oder 2,6-Naphthylen;
- Z: -O-; und
- D: eine geradkettige oder verzweigte Alkylengruppe mit 1 bis 20, insbesondere mit 1 bis 12 Kohlenstoffatomen oder ein gegebenenfalls mit Alkyl oder Alkoxy, insbesondere mit Methyl oder Methoxy, substituierter Cycloalkylrest mit 5 oder 6 Ringatomen;
bedeuten.

Ganz besonders bevorzugt sind Homopolymerzusammensetzungen mit wiederkehrenden Einheiten der allgemeinen Formel I, worin n = 0 ist und
- M¹ und S¹: die oben angegebenen Bedeutungen haben; und
- Ring B: unsubstituiertes oder gegebenenfalls mit Fluor, Chlor, Cyano, Alkyl oder Alkoxy substituiertes Phenylen, Pyridin-2,5-diyl, Pyrimidin-2,5-diyl oder Cyclohexan-1,4-diyl;
- Y²: eine einfache Kovalenzbindung, -CO-O- oder -O-OC-;
- m: 0 oder 1;
- Ring C: unsubstituiertes oder gegebenenfalls mit Fluor, Chlor, Cyano, Alkyl oder Alkoxy substituiertes Phenylen oder 1,4- oder 2,6-Naphthylen;
- n: 0;
- Z: -O-; und
- D: eine geradkettige oder verzweigte Alkylengruppe mit 1 bis 12 Kohlenstoffatomen;
bedeuten.

Solche ganz besonders bevorzugte Homopolymerzusammensetzungen sind beispielsweise:
Poly [1-[4-[(E)-2-methoxycarbonyl-vinyl]-phenoxycarbonyl]-1-methylethylen] ( Beispiel 1);
Poly [1-[4-[(E)-2-methoxycarbonyl-vinyl]-phenylaminocarbonyl]-1-methylethylen] ( Beispiel 1);
Poly [1-[2-[4-[(E)-2-methoxycarbonyl-vinyl]-phenoxy]-ethoxycarbonyl]-1-methyl-ethylen] ( Beispiele 2 und 3);
Poly [1-[6-[4-[2-methoxy-4-[(E)-2-methoxycarbonyl-vinyl]-phenoxycarbonyl]-phenoxy]-hexyloxycarbonyl]-1-methyl-ethylen] ( Beispiel 4);
Poly [1-[6-[4-[4-[(E)-2-methoxycarbonyl-vinyl]-phenoxycarbonyl]-phenoxy]-hexyloxycarbonyl]-1-methyl-ethylen] (Beispiel 4);
Poly [oxy-[4-[4-[4-[(E)-2-methoxycarbonyl-vinyl]-phenoxycarbonyl]-phenoxy]-butyl]-methyl-silylen] (Beispiel 5);
Poly [1-[2-[(E)-2-methoxycarbonyl-vinyl]-naphthalin-6-yloxycarbonyl]-1-methyl-ethylen] (Beispiel 8).

Die Polymere der Formel I zeichnen sich dadurch aus, dass sie einfach zugänglich sind. Die Methoden zur Herstellung sind dem Fachmann an sich bekannt.

Die Polymeren der Formel I können prinzipiell nach zwei unterschiedlichen Verfahren hergestellt werden. Neben der direkten Polymerisation vorgefertigter Monomere besteht die Möglichkeit der polymeranalogen Umsetzung von reaktionsfähigen Zimtsäurederivaten mit funktionellen Polymeren.

Zur direkten Polymerisation werden die Monomere und die Comonomere zunächst aus den einzelnen Bestandteilen getrennt zusammengesetzt. Die Bildung der Polymere erfolgt anschliessend in an sich bekannter Weise durch Einwirkung von UV-Strahlung oder Wärme oder durch Einwirkung radikalischer oder ionischer Katalysatoren. Beispiele für radikalische Initiatoren sind Kaliumperoxodisulfat, Dibenzoylperoxid, Azobisisobutyronitril oder Di-tert.-butylperoxid. Ionische Katalysatoren sind alkali-organische Verbindungen wie Phenyllithium oder Naphthylnatrium oder Lewissäuren wie BF₃, AlCl₃, SnCl₃ oder TiCl₄. Die Monomere können in Lösung, Suspension, Emulsion oder Substanz polymerisiert werden.

Im zweiten Verfahren kann ein Polymer der Formel I auch in einer polymeranalogen Reaktion aus einem vorgefertigten funktionellen Polymer und einem geeignet funktionalisierten Zimtsäurederivat hergestellt werden. Zur polymeranalogen Umsetzung eignen sich viele bekannte Verfahren wie zum Beispiel Veresterung, Umesterung, Amidierung oder die Veretherung.

Als vorteilhaft hat sich hier die Veretherung von Hydroxyzimtsäurederivaten mit Polyhydroxyalkylacrylaten bzw. Polyhydroxyalkylmethacrylaten in Lösung unter den Bedingungen der Mitsunobu-Reaktion erwiesen. Dabei kann man die Reaktion beispielsweise so führen, dass alle Hydroxylgruppen umgesetzt werden (Homopolymer) oder aber so, dass nach der Umsetzung noch freie Hydroxylgruppen am Polymer vorhanden sind, die dann in einer weiteren polymeranalogen Umsetzung weiter funktionalisiert werden können, wodurch sich Copolymere aufbauen lassen. Eine alternative Möglichkeit zur Herstellung von Copolymeren nach diesem Verfahren bietet die Verwendung von Gemischen aus verschiedenen Zimtsäurederivaten.

Die Zimtsäuren sind teilweise käuflich oder können nach literaturbekannten Verfahren wie etwa der Knoevenagel- oder der Wittig-Reaktion aus käuflichen Aldehyden oder aus Cyano-Verbindungen, durch vorherige Reduktion zu den entsprechenden Aldehyden, erhalten werden. Die Zimtsäureester oder Amide können dann aus den Zimtsäuren nach bekannten Veresterungsverfahren hergestellt werden.

Die Zimtsäureeinheiten der allgemeinen Formel I können nach dem Auftragen der Polymerschicht auf einen Träger durch Bestrahlung mit linear polarisiertem Licht dimerisiert werden. Durch die räumlich selektive Bestrahlung der Moleküleinheiten der Formel I können nun ganz bestimmte Bereiche einer Oberfläche ausgerichtet und durch die Dimerisierung gleichzeitig auch stabilisiert werden.

So kann zur Herstellung von Polymerorientierungsschichten in selektiv flächig begrenzten Bereichen beispielsweise zunächst eine Lösung des erhaltenen Polymermaterials hergestellt werden, welche in einer Spin-Coating-Apparatur auf einem, gegebenenfalls mit einer Elektrode beschichteten, Träger (z.B. mit Indium-Zinn-Oxid (ITO) beschichtete Glasplatte) aufgeschleudert wird, so dass homogene Schichten von 0,05 - 50 µm Dicke entstehen. Anschliessend können die zu orientierenden Bereiche z.B. mit einer Quecksilber-Hochdruck-Lampe, einer Xenonlampe oder einem gepulsten UV-Laser unter Verwendung eines Polarisators und gegebenenfalls einer Maske zur Abbildung von Strukturen belichtet werden. Die Belichtungsdauer ist abhängig von der Leistung der einzelnen Lampen und kann von wenigen Minuten bis zu mehreren Stunden variieren. Die Dimerisierung kann aber auch durch Bestrahlung der homogenen Schicht unter Verwendung von Filtern, die z.B. nur die für die Vernetzungsreaktion geeignete Strahlung hindurchlassen, erfolgen.

Die erfindungsgemässen Polymere werden durch die folgenden Beispiele weiter veranschaulicht. In den nachstehenden Beispielen bedeuten Tg die Glastemperatur, ε der molare dekadische Absorptionskoeffzient, G eine glasig erstarrte, C die kristalline, S die smektische, N die nematische und I die isotrope Phase, p die Anzahl der wiederkehrenden Enheiten, sodass sich Polymere mit einem Molekulargewicht M_{w} zwischen 1 000 und 5 000 000, vorzugsweise jedoch zwischen 5 000 und 2 000 000, besonders vorteilhaft jedoch zwischen 10 000 und 1 000 000 ergeben; w, w¹ und w² Molenbrüche der Comonomeren mit 0< w <1, 0 <w¹<1 und 0<w ²≤ 0,5 .

### Beispiel 1

### Poly[1-[4-[(E)-2-methoxycarbonyl-vinyl]-phenoxycarbonyl]-1-methylethylen]

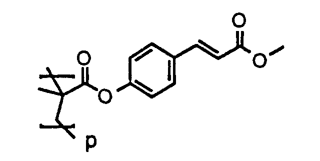

0,5 g (2,03 mmol) 2-Methyl-acrylsäure 4-[(E)-2-methoxycarbonyl-vinyl]-phenyl ester und 1,67 mg (0,01 mmol) 2,2'-Azo-bis-isobutyronitril (AIBN) wurden in 4,1 ml Tetrahydrofuran (THF) gelöst. Die Lösung wurde 15 Minuten mit einem schwachen Argonstrom durchspült. Anschliessend wurde das Reaktionsgefäss luftdicht verschlossen und auf 60 °C erhitzt. Nach 24 Stunden wurde das Gefäss geöffnet, die Lösung mit 4 ml THF verdünnt und unter starkem Rühren bei Raumtemperatur in 800 ml Diethylether getropft. Das ausgefallene Polymer wurde abfiltriert und bei 60 °C im Wasserstrahlvakuum getrocknet. Zur weiteren Reinigung wurde das Polymer in 10 ml Dichlormethan gelöst und erneut in 800 ml Diethylether ausgefällt. Dieser Vorgang wurde solange wiederholt, bis dünnschichtchromatographisch kein Monomer mehr nachweisbar war. Filtrieren und Trocknen bei 60 °C im Vakuum ergaben 0,37 g Poly [1-[4-[(E)-2-methoxycarbonyl-vinyl]-phenoxycarbonyl]-1-methylethylen] als weisses Pulver mit einer Glastufe bei Tg = 145 °C und einem Absorptionsmaximum von λ_{max.} (in CH₂Cl₂) = 275,2 nm (ε = 21430 l/mol cm).

Der als Ausgangsmaterial verwendete 2-Methyl-acrylsäure 4-[(E)-2-methoxycarbonyl-vinyl]-phenyl ester wurde nach folgendem Verfahren hergestellt:

### 3-(4-Hydroxyphenyl)-acrylsäure methyl ester

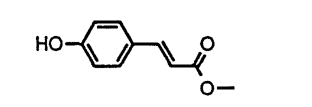

51,2 g (312 mmol) p-Cumarsäure wurden in 330 ml Methanol gelöst und mit 10 ml konzentrierter Schwefelsäure versetzt. Die Lösung wurde 2 Stunden unter Rückfluss erhitzt. Anschliessend wurde die Hauptmenge des Methanols (ca. 200 ml) abdestilliert und der vebliebende Rest auf 1,3 l Eiswasser gegossen. Der ausgefallene Ester wurde abgesaugt und nacheinander mit kaltem Wasser, mit wenig kalter NaHCO₃-Lösung und wieder mit kaltem Wasser gewaschen. Trocknung bei 50 °C im Wasserstrahlvakuum ergaben 51,1 g 3-(4-Hydroxyphenyl)-acrylsäure methyl ester in Form eines leicht bräunlich gefärbten Pulvers.

### 2-Methyl-acrylsäure 4-[(E)-2-methoxycarbonyl-vinyl]-phenyl ester

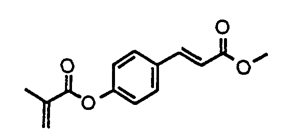

10,5 g (59 mmol) 3-(4-Hydroxy-phenyl)-acrylsäure methyl ester wurden in 170 ml Tetrahydrofuran gelöst und nacheinander mit 9,1 ml (65 mmol) Triethylamin und 0,079 g (0,65 mmol) 4-Dimethylamino-pyridin (DMAP) versetzt. Zu der auf ca. 15 °C abgekühlten Lösung wurde im Laufe von 30 Minuten 6,8 g (65 mmol) Methacrylsäurechlorid getropft. Der Reaktionsansatz wurde bei Raumtemperatur über Nacht gerührt und anschliessend über eine dünne Kieselgelschicht filtriert. Die Kieselgelschicht wurde gründlich mit THF gespült. Nach Abdampfen des Lösungsmittels wurde das Rohprodukt aus ca. 200 ml Ethanol umkristallisiert. Es wurde abfiltriert, getrocknet und ein weiteres Mal aus Ethanol umkristallisiert. Abfiltrieren und Trocknen bei 50 °C im Wasserstrahlvakuum ergaben 10,3 g 2-Methyl-acrylsäure 4-[(E)-2-methoxycarbonyl-vinyl]-phenyl ester als weisse Kristalle mit einem Schmelzpunkt von 76 - 80 °C und einem Absorptionsmaximum von λ_{max.} (in CH₂Cl₂) = 281,7 nm (ε = 24290 l/mol cm).

In analoger Weise lassen sich folgende Polymere synthetisieren:
Poly [1-[4-[(E)-2-ethoxycarbonyl-vinyl]-phenoxycarbonyl]-1-methylethylen];
Poly [1-[4-[(E)-2-propoxycarbonyl-vinyl]-phenoxycarbonyl]-1-methylethylen];
Poly [1-[4-[(E)-2-butoxycarbonyl-vinyl]-phenoxycarbonyl]-1-methylethylen];
Poly [1-[4-[(E)-2-hexyloxycarbonyl-vinyl]-phenoxycarbonyl]-1-methylethylen];
Poly [1-[4-[(E)-2-methoxycarbonyl-vinyl]-phenoxycarbonyl]-1-methylethylen-co-1-[4-[(E)-2-hexyloxycarbonyl-vinyl]-phenoxycarbonyl]-1-methylethylen];
Poly [1-[4-[(E)-2-methoxycarbonyl-vinyl]-phenoxycarbonyl]-1-methylethylen-co-1-[4-[(E)-2-octyloxycarbonyl-vinyl]-phenoxycarbonyl]-1-methylethylen];
Poly [1-[4-[(E)-2-ethoxycarbonyl-vinyl]-phenoxycarbonyl]-1-methylethylen-co-1-[4-[(E)-2-pentyloxycarbonyl-vinyl]-phenoxycarbonyl]-1-methylethylen];
Poly [1-[4-[(E)-2-methoxycarbonyl-vinyl]-phenylaminocarbonyl]-1-methylethylen], Tg = 207 °C;
Poly [1-[4-[(E)-2-butoxycarbonyl-vinyl]-phenylaminocarbonyl]-1-methylethylen];
Poly [1-[4-[(E)-2-methoxycarbonyl-vinyl]-phenylaminocarbonyl]-1-methylethylen-co-1-[4-[(E)-2-pentyloxycarbonyl-vinyl]-phenylaminocarbonyl]-1-methyl-ethylen];
Poly [1-[4-[(E)-2-methylaminocarbonyl-vinyl]-phenoxycarbonyl]-1-methylethylen];
Poly [1-[4-[(E)-2-dimethylaminocarbonyl-vinyl]-phenoxycarbonyl]-1-methyl-ethylen];
Poly [1-[4-[4-[(E)-2-methoxycarbonyl-vinyl]-phenyl]-phenoxycarbonyl]-1-methyl-ethylen];
Poly [1-[4-[4-[(E)-2-methoxycarbonyl-vinyl]-phenyl]-cyclohexyloxycarbonyl]- 1-methyl-ethylen];
Poly [1-[4-[4-[(E)-2-methoxycarbonyl-vinyl]-phenyl]-cyclohexylmethoxycarbonyl]- 1-methyl-ethylen].

### Beispiel 2:

### Poly [1-[2-[4-[(E)-2-methoxycarbonyl-vinyl]-phenoxy]-ethoxycarbonyl]-1-methyl-ethylen]

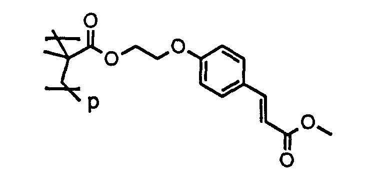

0,555 g (1,91 mmol) 2-Methyl-acrylsäure 2-[4-[(E)-2-methoxycarbonylvinyl]-phenoxy]-ethyl ester und 3,14 mg (0,019 mmol) 2,2'-Azo-bis-isobutyronitril (AIBN) wurden in 3,8 ml Tetrahydrofuran (THF) gelöst. Die Lösung wurde 15 Minuten mit einem schwachen Argonstrom durchspült. Anschliessend wurde das Reaktionsgefäss luftdicht verschlossen und auf 55 °C erhitzt. Nach 24 Stunden wurde das Gefäss geöffnet, die Lösung mit 4 ml THF verdünnt und unter starkem Rühren bei Raumtemperatur in 800 ml Diethylether getropft. Das ausgefallene Polymer wurde abfiltriert und bei 60 °C im Wasserstrahlvakuum getrocknet. Zur weiteren Reinigung wurde das Polymer in 10 ml Dichlormethan gelöst und wiederum in 800 ml Diethylether ausgefällt. Dieser Vorgang wurde solange wiederholt, bis dünnschichtchromatographisch kein Monomer mehr nachweisbar war. Filtrieren und Trocknen bei 60 °C im Vakuum ergaben 0,34 g Poly [1-[2-[4-[(E)-2-methoxycarbonyl-vinyl]-phenoxy]-ethoxycarbonyl]-1-methyl-ethylen] als weisses Pulver mit einer Glastufe bei Tg = 92 °C und einem Absorptionsmaximum von λ_{max.} (in CH₂Cl₂) = 296,3 nm (ε = 21680 l/mol cm).

Der als Ausgangsmaterial verwendete 2-Methyl-acrylsäure 2-[4-[(E)-2-methoxycarbonyl-vinyl]-phenoxy]-ethyl ester wurde nach folgendem Verfahren hergestellt:

### (E)-3-[4-[2-Hydroxyethoxy]-phenyl]-acrylsäure methyl ester

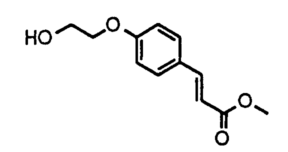

30 g (168 mmol) 3-(4-Hydroxyphenyl)-acrylsäure methyl ester (Beispiel 1), 29 g (210 mmol) wasserfreies K₂CO₃ und eine Spatelspitze KJ wurden in 200 ml Dimethylformamid vorgelegt. Unter Rühren wurden bei 85 °C innerhalb von 5 Minuten 14,91 g (185 mmol) 2-Chlorethanol zugetropft. Der Ansatz wurde noch 3 Tage bei 85 °C gerührt. Anschliessend wurden die Salze abfiltriert und das Filtrat im Wasserstrahlvakuum bis zur Trockne eingeengt. Nach Umkristallisation aus i-Propanol erhielt man 16,1 g (E)-3-[4-[2-Hydroxyethoxy]-phenyl]-acrylsäure methyl ester in Form weisser Kristalle.

### 2-Methyl-acrylsäure 2-[4-[(E)-2-methoxycarbonyl-vinyl]-phenoxy]ethyl ester

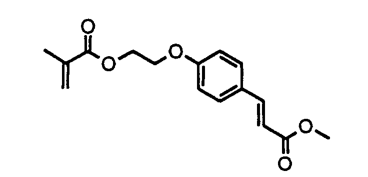

Zu einer Lösung von 6 g (27 mmol) (E)-3-[4-[2-Hydroxyethoxy]-phenyl]-acrylsäure methyl ester, 5,85 g (28,3 mmol) N,N'-Dicyclohexylcarbodiimid (DCC) und 0,37 g (3 mmol) 4-Dimethylamino-pyridin in 80 ml Tetrahydrofuran (THF) wurden 2,56 g (30 mmol) Methacrylsäure in 10 ml THF langsam zugetropft. Der Ansatz wurde über Nacht bei Raumtemperatur gerührt. Zur Vervollständigung der Reaktion wurden dann zunächst noch einmal 1,46 g (7,1 mmol) DCC und nach einer Stunde Rühren weitere 0,5 g (5,9 mmol) Methacrylsäure zugegeben. Der Ansatz wurde noch 24 Stunden weitergerührt, abfiltriert und das Filtrat je 3 mal mit 200 ml 5 %iger Essigsäure und 200 ml Wasser ausgeschüttelt. Die Etherphase wurde über Na₂SO₄ getrocknet, eingedampft und der Rückstand aus Cyclohexan umkristallisiert. Anschliessend wurde das noch leicht verunreinigte Produkt über eine dünne Kieselgelschicht filtriert (Laufmittel: Diethylether / Hexan = 1:1). Dies ergab 8,3 g 2-Methylacrylsäure 2-[4-[(E)-2-methoxycarbonyl-vinyl]-phenoxy]-ethyl ester in Form eines weissen Pulvers mit einem Schmelzpunkt von 81 - 82 °C und einem Absorptionsmaximum von λ_{max.} (in CH₂Cl₂) = 306,5 nm (ε = 23675 l/mol cm).

In analoger Weise lassen sich folgende Polymere synthetisieren:
Poly [1-[2-[4-[(E)-2-ethoxycarbonyl-vinyl]-phenoxy]-ethoxycarbonyl]-1-methyl-ethylen];
Poly [1-methyl-1-[2-[4-[(E)-2-propoxycarbonyl-vinyl]-phenoxy]-ethoxycarbonyl]-ethylen], T_{g} = 64 °C;
Poly [1-[2-[3-[(E)-2-methoxycarbonyl-vinyl]-phenoxy]-ethoxycarbonyl]-1-methyl-ethylen];
Poly [1-[2-[6-methoxy-3-[(E)-2-methoxycarbonyl-vinyl]-phenoxy]-ethoxycarbonyl]- 1-methyl-ethylen];
Poly [1-[2-[2-methoxy-4-[(E)-2-methoxycarbonyl-vinyl]-phenoxy]-methoxycarbonyl]-1-methyl-ethylen];
Poly [1-[2-[2-ethoxy-4-[(E)-2-methoxycarbonyl-vinyl]-phenoxy]-methoxycarbonyl]- 1-methyl-ethylen];
Poly [1-[2-[2-fluoro-4-[(E)-2-methoxycarbonyl-vinyl]-phenoxy]-methoxycarbonyl]-1-methyl-ethylen];
Poly [1-[2-[4-[(E)-2-methoxycarbonyl-vinyl]-2-methyl-phenoxy]-methoxycarbonyl]- 1-methyl-ethylen];
Poly [1-[1,1-dimethyl-2-[4-[(E)-2-methoxycarbonyl-vinyl]-phenoxy]-ethoxycarbonyl]-1-methyl-ethylen];
Poly [1-[2-[4-[(E)-2-methoxycarbonyl-vinyl]-phenoxy]-ethoxycarbonyl]-1-methyl-ethylen-co-1-methyl-1-[2-[4-[(E)-2-propoxycarbonyl-vinyl]-phenoxy]-ethoxycarbonyl]-ethylen] (9:1), Tg = 88 °C;
Poly [1-[2-[4-[(E)-2-methoxycarbonyl-vinyl]-phenoxy]-ethoxycarbonyl]-1-methyl-ethylen-co-1-methyl-1-[2-[4-[(E)-2-propoxycarbonyl-vinyl]-phenoxy]-ethoxycarbonyl]-ethylen] (1:1), Tg = 76 °C;
Poly [1-[2-[4-[(E)-2-methoxycarbonyl-vinyl]-phenoxy]-ethoxycarbonyl]-1-methyl-ethylen-co-1-[2-[4-[(E)-2-butoxycarbonyl-vinyl]-phenoxy]-ethoxycarbonyl]- 1-methyl-ethylen];
Poly [1-[2-[4-[(E)-2-methoxycarbonyl-vinyl]-phenoxy]-ethoxycarbonyl]-1-methyl-ethylen-co-1-[2-[4-[(E)-2-(2-methyl-butoxy)-carbonyl-vinyl]-phenoxy]-ethoxycarbonyl]- 1-methyl-ethylen];
Poly [1-[2-[4-[(E)-2-methoxycarbonyl-vinyl]-phenoxy]-ethoxycarbonyl]-1 -methyl-ethylen-co-1-[2-[4-[(E)-2-octyloxycarbonyl-vinyl]-phenoxy]-ethoxycarbonyl]-1-methyl-ethylen];
Poly [1-[2-[4-[(E)-2-methoxycarbonyl-vinyl]-phenoxy]-ethoxycarbonyl]-1-methyl-ethylen-co-1-[2-[4-[(E)-2-dodecyloxycarbonyl-vinyl]-phenoxy]-ethoxycarbonyl]- 1-methyl-ethylen];
Poly [1-[2-[4-[(E)-2-methoxycarbonyl-vinyl]-2-methyl-phenoxy]-ethoxycarbonyl]- 1-methyl-ethylen];
Poly [1-[2-[2-methoxy-4-[(E)-2-methoxycarbonyl-vinyl]-phenoxy]-ethoxycarbonyl]-1-methyl-ethylen];
Poly [1-[2-[4-[4-[(E)-2-methoxycarbonyl-vinyl]-phenyl]-phenoxy]-ethoxycarbonyl]- 1-methyl-ethylen];
Poly [1-[6-[4-[4-[(E)-2-methoxycarbonyl-vinyl]-phenyl]-phenoxy]-hexyloxycarbonyl]- 1-methyl-ethylen];
Poly [1-[2-[4-[4-[(E)-2-methoxycarbonyl-vinyl]-phenyl]-cyclohexyloxy]-ethoxycarbonyl]-1-methyl-ethylen];
Poly [1-[4-[4-[4-[(E)-2-methoxycarbonyl-vinyl]-phenyl]-cyclohexyloxy]-butoxycarbonyl]-1-methyl-ethylen];
Poly [1-[2-[4-[5-[(E)-2-methoxycarbonyl-vinyl]-pyridin-2-yl]-phenoxy]-ethoxycarbonyl]- 1-methyl-ethylen];
Poly [1-[2-[2-[4-[(E)-2-methoxycarbonyl-vinyl]-phenyl]-pyrimidin-5-yloxy]-ethoxycarbonyl]-1-methyl-ethylen];
Poly [1-[2-[6-[(E)-2-methoxycarbonyl-vinyl]-naphthalin-2-yloxy]-ethoxycarbonyl]-1-methyl-ethylen];
Poly [1-[2-[4-[(E)-2-methoxycarbonyl-vinyl]-naphthalin-1-yloxy]-ethoxycarbonyl]- 1-methyl-ethylen];
Poly [1-[8-[4-[(E)-2-methoxycarbonyl-vinyl]-naphthalin-1-yloxy]-octyloxycarbonyl]- 1-methyl-ethylen].

### Beispiel 3:

### Poly [1-[2-[4-[(E)-2-methoxycarbonyl-vinyl]-phenoxy]-ethoxycarbonyl]-1-methyl-ethylen]

### durch polymeranaloge Veretherung nach Mitsunobu

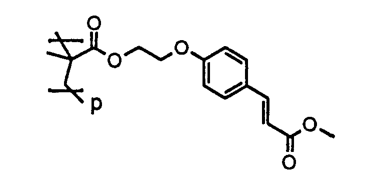

2,5 g (4,05 mmol) einer Stammlösung von Poly (2-hydroxy-ethyl methacrylat) (21 Gew. % in DMA) wurden unter Argon mit 7,5 ml Dimethylacetamid (DMA) verdünnt. Unter Rühren wurden bei Raumtemperatur 2,32 g (8,8 mmol) Triphenylphosphin und 1,44 g (8,1 mmol) 3-(4-Hydroxyphenyl)-acrylsäure methyl ester im Reaktionsansatz gelöst. Die Lösung wurde auf 0 °C abgekühlt. Innerhalb von 4 Stunden wurden 1,4 ml (8,8 mmol) Azodicarbonsäure-diäthylester (DEAD) zugetropft. Der Reaktionsansatz wurde noch 15 Minuten bei 0 °C belassen und dann nach Entfernung des Eisbades 15 Stunden bei Raumtemperatur gerührt. Die Reaktionsmischung wurde dann unter starkem Rühren in ca. 900 ml Diethylether getropft. Das ausgefallene Polymer wurde abfiltriert und bei 60 °C im Wasserstrahlvakuum getrocknet. Zur Reinigung wurde der Rückstand in 10 ml Dichlormethan gelöst und erneut in Diethylether ausgefällt. Dieser Vorgang wurde solange wiederholt, bis dünnschichtchromatographisch kein Monomer mehr nachweisbar war. Filtrieren und Trocknen bei 60 °C im Vakuum ergaben 0,93 g Poly [1-[2-[4-[(E)-2-methoxycarbonyl-vinyl]-phenoxy]-ethoxycarbonyl]-1-methyl-ethylen] als weisses Pulver mit einer Glastufe bei Tg = 88 °C und einem Absorptionsmaximum von λ_{max.} (in CH₂Cl₂) = 296,6 nm (ε = 20610 l/mol cm).

In analoger Weise lassen sich folgende Polymere synthetisieren:
Poly [1-[2-[4-[(E)-2-methoxycarbonyl-vinyl]-phenoxy]-ethoxycarbonyl]-1-methyl-ethylen-co-1-[2-hydroxy-ethoxycarbonyl]-1-methyl-ethylen], Tg = 90 °C;
Poly [1-[2-[4-[(E)-2-ethoxycarbonyl-vinyl]-phenoxy]-ethoxycarbonyl]-1 -methyl-ethylen-co- 1-[2-hydroxy-ethoxycarbonyl]- 1-methyl-ethylen];
Poly [1-[2-[3-[(E)-2-methoxycarbonyl-vinyl]-phenoxy]-ethoxycarbonyl]-1-methyl-ethylen-co-1-[2-hydroxy-ethoxycarbonyl]-1-methyl-ethylen];
Poly [1-[2-[3-[(E)-2-methoxycarbonyl-vinyl]-phenoxy]-ethoxycarbonyl]-1-methyl-ethylen-co-1-[2-hydroxy-ethoxycarbonyl]-1-methyl-ethylen] (4:1), Tg = 73 °C;
Poly [1-[2-[6-methoxy-3-[(E)-2-methoxycarbonyl-vinyl]-phenoxy]-ethoxycarbonyl]-1-methyl-ethylen-co-1-[2-hydroxy-ethoxycarbonyl]-1-methylethylen];
Poly [1-[2-[2-methoxy-4-[(E)-2-methoxycarbonyl-vinyl]-phenoxy]-ethoxycarbonyl]-1-methyl-ethylen], Tg = 84 °C;
Poly [1-[4-[2-methoxy-4-[(E)-2-methoxycarbonyl-vinyl]-phenoxy]-butoxycarbonyl]-1-methyl-ethylen], Tg = 60 °C;
Poly [1-[2-[2-methoxy-4-[(E)-2-methoxycarbonyl-vinyl]-phenoxy]-methoxycarbonyl]-1-methyl-ethylen-co-1-[2-hydroxy-ethoxycarbonyl]-1-methylethylen];
Poly [1-[2-[2-ethoxy-4-[(E)-2-methoxycarbonyl-vinyl]-phenoxy]-methoxycarbonyl]-1-methyl-ethylen-co-1-[2-hydroxy-ethoxycarbonyl]-1-methylethylen];
Poly [1-[2-[2-fluoro-4-[(E)-2-methoxycarbonyl-vinyl]-phenoxy]-methoxycarbonyl]-1-methyl-ethylen-co-1-[2-hydroxy-ethoxycarbonyl]-1-methyl-ethylen];
Poly [1-[2-[4-[(E)-2-methoxycarbonyl-vinyl]-2-methyl-phenoxy]-methoxycarbonyl]-1-methyl-ethylen-co-1-[2-hydroxy-ethoxycarbonyl]-1-methyl-ethylen];
Poly [1-[1,1-dimethyl-2-[4-[(E)-2-methoxycarbonyl-vinyl]-phenoxy]-ethoxycarbonyl]-1-methyl-ethylen-co-1-[2-hydroxy-ethoxycarbonyl]-1-methylethylen];
Poly [1-[2-[4-[(E)-2-methoxycarbonyl-vinyl]-phenoxy]-ethoxycarbonyl]-1-methyl-ethylen-co-1-[2-[4-[(E)-2-propoxycarbonyl-vinyl]-phenoxy]-ethoxycarbonyl]-1-methyl-ethylen-co-1-[2-hydroxy-ethoxycarbonyl]-1-methyl-ethylen] (15:3:7), Tg = 101 °C;
Poly [1-[2-[4-[(E)-2-methoxycarbonyl-vinyl]-phenoxy]-ethoxycarbonyl]-1-methyl-ethylen-co-1-[2-[4-[(E)-2-propoxycarbonyl-vinyl]-phenoxy]-ethoxycarbonyl]-1-methyl-ethylen-co-1-[2-hydroxy-ethoxycarbonyl]-1-methyl-ethylen] (11:4:3), Tg = 94 °C;
Poly [1-[2-[4-[(E)-2-methoxycarbonyl-vinyl]-phenoxy]-ethoxycarbonyl]-1-methyl-ethylen-co-1-[2-[4-[(E)-2-butoxycarbonyl-vinyl]-phenoxy]-ethoxycarbonyl]-1-methyl-ethylen-co-1-[2-hydroxy-ethoxycarbonyl]-1-methyl-ethylen];
Poly [1-[2-[4-[(E)-2-methoxycarbonyl-vinyl]-phenoxy]-ethoxycarbonyl]-1-methyl-ethylen-co-1-[2-[4-[(E)-2-(2-methyl-butoxy)-carbonyl-vinyl]-phenoxy]-ethoxycarbonyl]-1-methyl-ethylen-co-1-[2-hydroxy-ethoxycarbonyl]-1-methylethylen];
Poly [1-[2-[4-[(E)-2-methoxycarbonyl-vinyl]-phenoxy]-ethoxycarbonyl]-1-methyl-ethylen-co-1-[2-[4-[(E)-2-octyloxycarbonyl-vinyl]-phenoxy]-ethoxycarbonyl]-1-methyl-ethylen-co-1-[2-hydroxy-ethoxycarbonyl]-1-methyl-ethylen];
Poly [1-[2-[4-[(E)-2-methoxycarbonyl-vinyl]-phenoxy]-ethoxycarbonyl]-1-methyl-ethylen-co-1-[2-[4-[(E)-2-dodecyloxycarbonyl-vinyl]-phenoxy]-ethoxycarbonyl]-1-methyl-ethylen-co-1-[2-hydroxy-ethoxycarbonyl]-1-methylethylen];
Poly [1-[2-[4-[(E)-2-methoxycarbonyl-vinyl]-2-methyl-phenoxy]-ethoxycarbonyl]-1-methyl-ethylen-co-1-[2-hydroxy-ethoxycarbonyl]-1-methyl-ethylen];
Poly [1-[2-[2-methoxy-4-[(E)-2-methoxycarbonyl-vinyl]-phenoxy]-ethoxycarbonyl]-1-methyl-ethylen-co-1-[2-hydroxy-ethoxycarbonyl]-1-methyl-ethylen];
Poly [1-[2-[4-[4-[(E)-2-methoxycarbonyl-vinyl]-phenyl]-phenoxy]-ethoxycarbonyl]- 1-methyl-ethylen-co- 1-[2-hydroxy-ethoxycarbonyl]- 1-methyl-ethylen];
Poly [1-[6-[4-[4-[(E)-2-methoxycarbonyl-vinyl]-phenyl]-phenoxy]-hexyloxycarbonyl]- 1-methyl-ethylen];
Poly [1-[2-[4-[4-[(E)-2-methoxycarbonyl-vinyl]-phenyl]-cyclohexyloxy]-ethoxycarbonyl]-1-methyl-ethylen-co-1-[2-hydroxy-ethoxycarbonyl]-1-methylethylen];
Poly [1-[4-[4-[4-[(E)-2-methoxycarbonyl-vinyl]-phenyl]-cyclohexyloxy]-butoxycarbonyl]-1-methyl-ethylen-co-1-[2-hydroxy-ethoxycarbonyl]-1-methylethylen];
Poly [1-[2-[4-[5-[(E)-2-methoxycarbonyl-vinyl]-pyridin-2-yl]-phenoxy]-ethoxycarbonyl]-1-methyl-ethylen-co-1-[2-hydroxy-ethoxycarbonyl]-1-methylethylen];
Poly [1-[2-[2-[4-[(E)-2-methoxycarbonyl-vinyl]-phenyl]-pyrimidin-5-yloxy]-ethoxycarbonyl]-1-methyl-ethylen-co-1-[2-hydroxy-ethoxycarbonyl]-1-methylethylen];
Poly [1-[2-[6-[(E)-2-methoxycarbonyl-vinyl]-naphthalin-2-yloxy]-ethoxycarbonyl]- 1-methyl-ethylen-co- 1-[2-hydroxy-ethoxycarbonyl]- 1-methyl-ethylen];
Poly [1-[2-[4-[(E)-2-methoxycarbonyl-vinyl]-naphthalin-1-yloxy]-ethoxycarbonyl]- 1-methyl-ethylen-co- 1-[2-hydroxy-ethoxycarbonyl]- 1-methyl-ethylen];
Poly [1-[8-[4-[(E)-2-methoxycarbonyl-vinyl]-naphthalin-1-yloxy]-octyloxycarbonyl]- 1-methyl-ethylen-co- 1-[2-hydroxy-ethoxycarbonyl]- 1-methyl-ethylen].

### Beispiel 4:

### Poly [1-[6-[4-[2-methoxy-4-[(E)-2-methoxycarbonyl-vinyl]-phenoxycarbonyl]-phenoxy]-hexyloxycarbonyl]-1-methyl-ethylen]

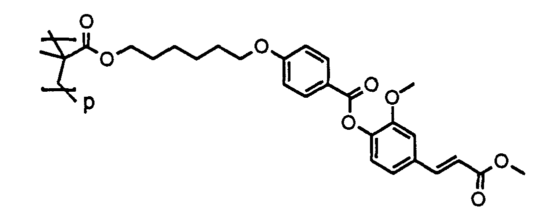

2,5 g (5 mmol) 4-[6-(2-Methyl-acryloyloxy)-hexyloxy]-benzoesäure 2-methoxy-4-[(E)-2-methoxycarbonyl-vinyl]-phenyl ester und 8,2 mg (0,05 mmol) 2,2'-Azo-bis-isobutyronitril wurden in 10 ml Tetrahydrofuran (THF) gelöst. Die Lösung wurde 30 Minuten mit einem schwachen Argonstrom durchspült. Anschliessend wurde das Reaktionsgefäss luftdicht verschlossen und auf 55 °C erhitzt. Nach 24 Stunden wurde das Gefäss geöffnet, die Lösung mit 8 ml THF verdünnt und unter starkem Rühren bei Raumtemperatur in 1,6 Methanol getropft. Das ausgefallene Polymer wurde abfiltriert und bei 50 °C im Wasserstrahlvakuum getrocknet. Zur weiteren Reinigung wurde das Polymer in ca. 25 ml Dichlormethan gelöst und erneut in 1,75 l Methanol gefällt. Dieser Vorgang wurde solange wiederholt, bis dünnschichtchromatographisch kein Monomer mehr nachweisbar war. Filtrieren und Trocknen bei 50 °C im Wasserstrahlvakuum ergaben 2,14 g Poly [1-[6-[4-[2-methoxy-4-[(E)-2-methoxycarbonyl-vinyl]-phenoxycarbonyl]-phenoxy]-hexyloxycarbonyl]-1-methylethylen] mit einem Molekulargewicht M_{w} von 769 000 (Gelpermeationschromatographie: THF, 35 °C, Polystyrolstandard), mit einer Glastufe bei Tg = 74 °C und einem Absorptionsmaximum von λ_{max.} (in CH₂Cl₂) = 277,0 nm (ε = 31575 l/mol cm).

Der als Ausgangsmaterial verwendete 4-[6-(2-Methyl-acryloyloxy)-hexyloxy]-benzoesäure 2-methoxy-4-[(E)-2-methoxycarbonyl-vinyl]-phenyl ester wurde nach folgendem Verfahren hergestellt:

### 4-(6-Hydroxy-hexyloxy)-benzoesäure

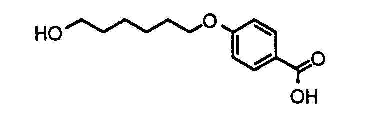

229,2 g (1,66 mol) p-Hydroxy-benzoesäure wurden in 600 ml Methanol gelöst und bei 60 °C innerhalb von 10 Minuten mit einer Lösung aus 151 g (3,77 mol) NaOH in 480 ml H₂O versetzt. Zu dieser Lösung wurden 271,2 g (1,99 mol) 6-Chlor-hexanol langsam zugetropft. Schliesslich wurden noch 0,75 g Kaliumiodid zugegeben und der Ansatz 60 Stunden unter Rückfluss gekocht. Zur Aufarbeitung wurde die gelbe Lösung in 31 H₂O gegossen und mit 10 %iger HCl (ca. 600 ml) versetzt bis ein pH-Wert von 1 erreicht war. Die milchige Suspension wurde über eine grosse Nutsche filtriert. Der Rückstand wurde trockengesaugt und zweimal aus ca. 1,51 Ethanol umkristallisiert. Dies ergab 229,6 g 4-(6-Hydroxy-hexyloxy)-benzoesäure als feines weisses Pulver; Smp. 136 - 141 °C.

### 4-[6-(2-Methyl-acryloyloxy)-hexyloxy]-benzoesäure

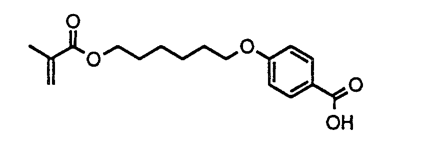

71,5 g (0,3 mol) 4-(6-Hydroxy-hexyloxy)-benzoesäure und 101,5 g (1,18 mol) Methacrylsäure wurden in 950 ml Chloroform gelöst. Nach Zugabe von 7,2 g (0,07 mol) Hydrochinon und 7,2 g (0,04 mol) p-Toluolsulfonsäure wurde der Ansatz 48 Stunden unter Rückfluss am Wasserabscheider gekocht. Die klare braune Lösung wurde anschliessend eingedampft, der Rückstand in 1,5 l Diethylether aufgenommen, filtriert und fünfmal mit je 300 ml H₂O geschüttelt. Die organische Phase wurde über Na₂SO₄ getrocknet, eingedampft und der Rückstand zweimal aus Methanol umkristallisiert. Nach Trocknung im Wasserstrahlvakuum bei 40 °C verblieben 47,33 g 4-[6-(2-Methyl-acryloyloxy)-hexyloxy]-benzoesäure als weisses Pulver; Smp. 83 °C.

### 4-Hydroxy-3-methoxy-zimtsäure methyl ester

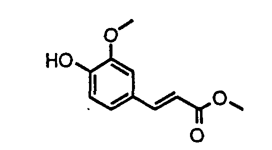

Die Darstellung erfolgte analog Beispiel 1 aus 25 g (0,129 mol) 4-Hydroxy-3-methoxy-zimtsäure und 180 ml Methanol mit konzentrierter Schwefelsäure als Katalysator. Zur Reinigung wurde an Kieselgel mit Dichlormethan / Diethylether (19:1) chromatographiert. Dies ergab 21,78 g 4-Hydroxy-3-methoxy-zimtsäure methyl ester als hellgelbes Öl.

### 4-[6-(2-Methyl-acryloyloxy)-hexyloxy]-benzoesäure 2-methoxy-4-[(E)-2-methoxycarbonyl-vinyl]-phenyl ester

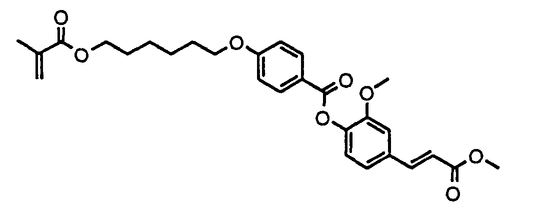

8,5 g (0,028 mol) 4-[6-(2-Methyl-acryloyloxy)-hexyloxy]-benzoesäure wurden mit 6 ml Thionylchlorid und 3 Tropfen DMF versetzt und 2 Stunden auf 90 °C erhitzt. Das überschüssige Thionylchlorid wurde zunächst im Wasserstrahl- und anschliessend im Hochvakuum vollständig entfernt. Das verbliebene Säurechlorid wurde in 20 ml Dichlormethan aufgenommen und bei 0 °C langsam zu einer Lösung aus 5,25 g (0,025 mol) 4-Hydroxy-3-methoxyzimtsäure methyl ester und 4,25 ml Triethylamin in 25 ml THF getropft. Der Ansatz wurde über Nacht bei Raumtemperatur gerührt, filtriert und das Filtrat bis zur Trockne eingedampft. Der Rückstand wurde durch Säulenchromatographie an Kieselgel mit Dichlormethan / Diethylether (19:1) und anschliessend durch Umkristallisation aus Ethanol/THF gereinigt. Es wurden 6,31 g 4-[6-(2-Methyl-acryloyloxy)-hexyloxy]-benzoesäure 2-methoxy-4-[(E)-2-methoxycarbonyl-vinyl]-phenyl ester als weisses Pulver isoliert; Smp. 92-94 °C.

In analoger Weise lassen sich folgende Polymere synthetisieren:
Poly [1-[6-[4-[4-[(E)-2-methoxycarbonyl-vinyl]-phenoxycarbonyl]-phenoxy]-hexyloxycarbonyl]-1-methyl-ethylen], Phasenfolge (°C): G 64 LC1 135 LC2 164 I;
Poly [1-[6-[4-[2-ethoxy-4-[(E)-2-methoxycarbonyl-vinyl]-phenoxycarbonyl]-phenoxy]-hexyloxycarbonyl]-1-methyl-ethylen], Tg = 64 °C;
Poly [1-[6-[4-[2-propoxy-4-[(E)-2-methoxycarbonyl-vinyl]-phenoxycarbonyl]-phenoxy]-hexyloxycarbonyl]-1-methyl-ethylen];
Poly [1-[6-[4-[2-butoxy-4-[(E)-2-methoxycarbonyl-vinyl)-phenoxycarbonyl)-phenoxy]-hexyloxycarbonyl]-1-methyl-ethylen];
Poly [1-[6-[4-[2-methyl-4-[(E)-2-methoxycarbonyl-vinyl]-phenoxycarbonyl]-phenoxy]-hexyloxycarbonyl]- 1-methyl-ethylen];
Poly [1-[4-[4-[2-methyl-4-[(E)-2-methoxycarbonyl-vinyl]-phenoxycarbonyl]-phenoxy]-butyloxycarbonyl]- 1-methyl-ethylen];
Poly [1-[2-[4-[2-methoxy-4-[(E)-2-methoxycarbonyl-vinyl]-phenoxycarbonyl]-phenoxy]-ethoxycarbonyl]-1-methyl-ethylen], Tg = 119 °C;
Poly [1-[3-[4-[2-methoxy-4-[(E)-2-methoxycarbonyl-vinyl]-phenoxycarbonyl]-phenoxy]-propoxycarbonyl]-1-methyl-ethylen], Tg = 102 °C;
Poly [1-[2-[2-methoxy-4-[4-[(E)-2-methoxycarbonyl-vinyl]-phenoxycarbonyl]-phenoxy]-ethoxycarbonyl]-1-methyl-ethylen];
Poly [1-[2-[2-methyl-4-[4-[(E)-2-methoxycarbonyl-vinyl]-phenoxycarbonyl]-phenoxy]-ethoxycarbonyl]-1-methyl-ethylen];
Poly [1-[6-[2-methoxy-4-[4-[(E)-2-methoxycarbonyl-vinyl]-phenoxycarbonyl]-phenoxy]-hexyloxycarbonyl]-1-methyl-ethylen];
Poly [1-[6-[2-methyl-4-[4-[(E)-2-methoxycarbonyl-vinyl)-phenoxycarbonyl)-phenoxy]-hexyloxycarbonyl]-1-methyl-ethylen];
Poly [1-[6-[4-[4-[2-methoxy-4-[(E)-2-methoxycarbonyl-vinyl]-phenoxycarbonyl]-phenyl]-phenoxy]-hexyloxycarbonyl]-1-methyl-ethylen];
Poly [1-[6-[4-[4-[2-methoxy-4-[(E)-2-methoxycarbonyl-vinyl]-phenoxycarbonyl]-cyclohexyl]-cyclohexyloxy]-hexyloxycarbonyl]-1-methyl-ethylen].

### Beispiel 5:

### Poly [oxy-[4-[4-[4-[(E)-2-methoxycarbonyl-vinyl]-phenoxycarbonyl]-phenoxy]-butyl]-methyl-silylen]

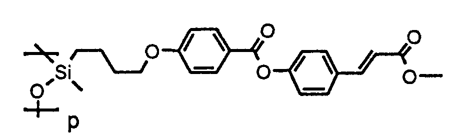

0,5 g (1,4 mmol) 4-(But-3-enyloxy)-benzoesäure 4-[(E)-2-methoxycarbonylvinyl]-phenyl ester und 0,071 g Poly(methyl-hydrogen-siloxan) wurden in 3 ml Toluol gelöst. Die Lösung wurde über ein Septum auf dem Reaktionsgefäss 10 Minuten lang mit einem schwachen Stickstoffstrom durchspült. Anschliessend wurden bei Raumtemperatur unter Rühren 8 µl einer Platin-divinyltetramethyldisiloxankomplex Lösung in das Reaktionsgemisch eingespritzt. Der Ansatz wurde 24 Stunden bei 55 °C gerührt. Dann wurde die Polymerlösung unter Rühren in 400 ml eisgekühltes n-Hexan getropft. Das ausgefallene Polymer wurde abgetrennt, getrocknet, in ca. 5 ml Toluol gelöst und in 400 ml Methanol ausgefällt. Dieser Vorgang wurde noch zweimal wiederholt. Nach Trocknen im Hochvakuum ergab dies 0,15 g Poly [oxy-[4-[4-[4-[(E)-2-methoxycarbonyl-vinyl]-phenoxycarbonyl]-phenoxy]-butyl]-methyl-silylen] als weisses Pulver mit der Phasenfolge (°C): G 35 S 194 I.

Der als Ausgangsmaterial verwendete 4-(But-3-enyloxy)-benzoesäure 4-[(E)-2-methoxycarbonyl-vinyl]-phenyl ester wurde nach folgendem Verfahren hergestellt:

### 4-(But-3-enyloxy)-benzoesäure

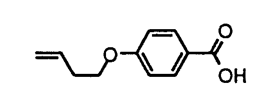

69,1 g (0,5 mol) p-Hydroxybenzoesäure wurden in 400 ml Ethanol gelöst und mit einer Lösung aus 56,1 g (1 mol) KOH in 250 ml Wasser versetzt. Der Reaktionsansatz wurde unter Rückfluss erhitzt. Dabei wurden 74,26 g (0,55 mol) 4-Brom-1-buten langsam zugetropft. Nach 5 Stunden wurde das Ethanol am Rotationsverdampfer entfernt. Die Wasserphase wurde mit NaOH auf einen pH-Wert von 10 gebracht und mehrfach mit Diethylether extrahiert. Die wässrige Phase wurde auf eine Mischung aus 46 ml konzentrierter HCl und 500 ml Eiswasser geschüttet. Die ausgefallene Säure wurde abfiltriert, mit wenig Wasser gewaschen und aus Methanol/Wasser (2:1) umkristallisiert. Nach dem Trocknen im Wasserstrahlvakuum bei 60 °C verblieben 40 g 4-(But-3-enyloxy)-benzoesäure als weisses Pulver; Phasenfolge (°C): C 120 N 141 I.

### 4-(But-3-enyloxy)-benzoesäure 4-[(E)-2-methoxycarbonyl-vinyl]-phenyl ester

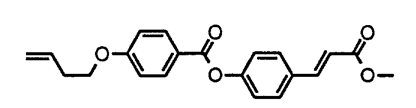

8,7 g (0,045 mol) 4-(But-3-enyloxy)-benzoesäure wurden mit 76 ml Thionylchlorid und 2 Tropfen DMF versetzt und 3,5 Stunden unter Rückfluss gekocht. Das überschüssige Thionylchlorid wurde zunächst im Wasserstrahl- und anschliessend im Hochvakuum vollständig entfernt. Das verbliebene Säurechlorid wurde in 10 ml Dichlormethan aufgenommen und bei 0 °C langsam zu einer Mischung aus 7,59 g (0,042 mol) 3-(4-Hydroxyphenyl)-acrylsäure methyl ester (Beispiel 1) und 6 ml Triethylamin in 40 ml Dichlormethan getropft. Der Ansatz wurde über Nacht bei Raumtemperatur gerührt. Die weissen Niederschläge im Reaktionsgemisch wurden durch Zugabe von Dichlormethan gelöst. Die organische Phase wurde mehrfach mit Wasser gewaschen, über Na₂SO₄ getrocknet und zur Trockne eingedampft. Zur Reinigung wurde der Rückstand mit Dichlormethan an Kieselgel chromatographiert und anschliessend aus Ethanol umkristallisiert. Es wurden 12,65 g 4-(But-3-enyloxy)-benzoesäure 4-[(E)-2-methoxycarbonyl-vinyl]-phenyl ester als weisses Pulver isoliert; Phasenfolge (°C): C 103 N 138 I.

In analoger Weise lassen sich folgende Polymere synthetisieren:
Poly [oxy-[4-[4-[2-methoxy-4-[(E)-2-methoxycarbonyl-vinyl]-phenoxycarbonyl]-phenoxy]-butyl]-methyl-silylen], Tg = 47 °C;
Poly [oxy-[4-[4-[2-fluoro-4-[(E)-2-methoxycarbonyl-vinyl]-phenoxycarbonyl]-phenoxy]-butyl]-methyl-silylen];
Poly [oxy-[6-[4-[2-methyl-4-[(E)-2-methoxycarbonyl-vinyl]-phenoxycarbonyl]-phenoxy]-hexyl]-methyl-silylen];
Poly [oxy-[4-[4-[4-[(E)-2-methoxycarbonyl-vinyl]-phenyl]-phenoxy]-butyl]-methyl-silylen];
Poly [oxy-[6-[4-[4-[(E)-2-methoxycarbonyl-vinyl]-phenyl]-cyclohexyloxy]-hexyl]-methyl-silylen];
Poly [oxy-[4-[4-[4-[(E)-2-methoxycarbonyl-vinyl]-phenoxycarbonyl]-phenoxy]-butyl]-methyl-silylen-co-oxy-[4-[4-[4-[(E)-2-hexyloxycarbonyl-vinyl]-phenoxycarbonyl]-phenoxy]-butyl]-methyl-silylen];
Poly [oxy-[4-[4-[4-[(E)-2-methoxycarbonyl-vinyl]-phenoxycarbonyl]-phenoxy]-butyl]-methyl-silylen-co-oxy-[6-[4-[4-[(E)-2-methoxycarbonyl-vinyl]-phenoxycarbonyl]-phenoxy]-hexyl]-methyl-silylen];
Poly [oxy-[4-[4-[4-[(E)-2-methoxycarbonyl-vinyl]-phenoxycarbonyl]-phenoxy]-butyl]-methyl-silylen-co-dimethyl-oxy-silylen];
Poly [oxy-[4-[4-[4-[(E)-2-methoxycarbonyl-vinyl]-phenoxycarbonyl]-phenoxy]-butyl]-methyl-silylen-co-oxy-[4-[4-[4-[(E)-2-butoxycarbonyl-vinyl]-phenoxycarbonyl]-phenoxy]-butyl]-methyl-silylen-co-dimethyl-oxy-silylen].

### Beispiel 6

### Poly[1-[3-[4-[4'-[(E)-2-methoxycarbonyl-vinyl]-biphenyl-4-yl]-cyclohexyl]-propoxycarbonyl]-1-methyl-ethylen]

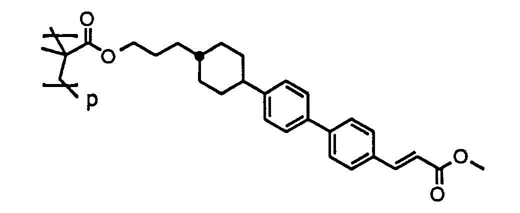

Die Polymerisation des trans-2-Methyl-acrylsäure 3-[4-[4'-[(E)-2-methoxycarbonyl-vinyl]-biphenyl-4-yl]-cyclohexyl]-propyl esters zu Poly [1-[3-[4-[4'-[(E)-2-methoxycarbonyl-vinyl]-biphenyl-4-yl]-cyclohexyl]-propoxycarbonyl]-1-methyl-ethylen] erfolgte analog Beispiel 1. Das Polymer besitzt folgende Phasenfolge (°C): G 156 C 208 I.

Der als Ausgangsmaterial verwendete trans-2-Methyl-acrylsäure 3-[4-[4'-[(E)-2-methoxycarbonyl-vinyl]-biphenyl-4-yl]-cyclohexyl]-propyl ester wurde nach folgendem Verfahren hergestellt:

### trans-4'-[4-(3-Hydroxy-propyl)-cyclohexyl]-biphenyl-4-carbonitril

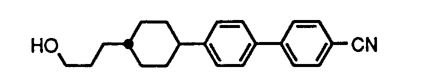

Zu einer Suspension von 1,39 g Natriumborhydrid in 30 ml Methanol / Ether (9:1) wurde bei 0 °C innerhalb von 5 Minuten eine Lösung von 12 g trans-4'-[4-(3-Oxo-propyl)-cyclohexyl]-biphenyl-4-carbonitril (Herstellung: Mol. Cryst. Liq. Cryst. 1985, Vol. 131, 327) in 100 ml Methanol / Ether (9:1) getropft. Nach 45 Minuten wurde nochmals 1 g Natriumborhydrid zugefügt. Nach einer weiteren Stunde wurde die Reaktion abgebrochen und zwischen Methylenchlorid und In Salzsäure verteilt. Hierauf wurde die organische Phase mehrmals mit Wasser gewaschen, über Magnesiumsulfat getrocknet, filtriert und eingedampft. Kristallisation aus Essigester / Methylenchlorid ergab 11,5 g trans-4'-[4-(3-Hydroxy-propyl)-cyclohexyl]-biphenyl-4-carbonitril als gelbliche Kristalle.

### trans-4'-[4-(3-Hydroxy-propyl)-cyclohexyl]-biphenyl-4-carboxaldehyd

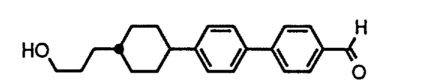

Zu einer Suspension von 11,5 g trans-4'-[4-(3-Hydroxy-propyl)-cyclohexyl]-biphenyl-4-carbonitril in 150 ml Toluol wurden innerhalb von 10 Minuten bei 0 °C 38,5 ml einer Diisobutylaluminiumhydrid Lösung (20% in Toluol) getropft. Dann wurde das Reaktionsgemisch langsam auf Raumtemperatur erwärmt und noch 3,5 Stunden reagieren gelassen. Anschliessend wurde langsam 1n Salzsäure zugetropft, 1 Stunde gerührt und hierauf das Reaktionsgemisch zwischen Wasser und Methylenchlorid verteilt. Danach wurde die organische Phase mehrmals mit Wasser gewaschen, über Magnesiumsulfat getrocknet, filtriert und eingedampft. Kristallisation aus Essigester / Methylenchlorid ergab 9,9 g trans-4'-[4-(3-Hydroxy-propyl)-cyclohexyl]-biphenyl-4-carboxaldehyd als leicht gelbe Kristalle.

### trans-4'-[4-[3-(2-Methyl-acryloyloxy)-propyl]-cyclohexyl]-biphenyl-4-carboxaldehyd

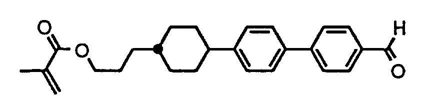

Zu einer Lösung von 2,9 ml Methacrylsäure in 30 ml Tetrahydrofuran wurden bei -25 °C zuerst 9,4 ml Triethylamin und dann 2,63 ml Methansulfonsäurechlorid getropft. Danach wurde weiterhin bei -25 °C während 1 Stunde gerührt und dann eine Lösung von 9,9 g trans-4'-[4-(3-Hydroxy-propyl)-cyclohexyl]-biphenyl-4-carboxaldehyd und 1,1 g 4-Dimethylamino-pyridin in 50 ml Tetrahydrofuran zugetropft. Danach wurde 2,5 Stunden bei 0 °C und dann 18 Stunden bei Raumtemperatur reagieren gelassen. Hierauf wurde das Reaktionsgemisch über Celite filtriert, das Filtrat zwischen Ether und Wasser verteilt, die Etherphase über Magnesiumsulfat getrocknet und eingedampft. Dies ergab 11,5 g rohen trans-4'-[4-[3-(2-Methyl-acryloyloxy)-propyl]-cyclohexyl]-biphenyl-4-carboxaldehyd in fester Form.

### trans-2-Methyl-acrylsäure 3-[4-[4'-[(E)-2-methoxycarbonyl-vinyl]-biphenyl-4-yl]-cyclohexyl]-propyl ester

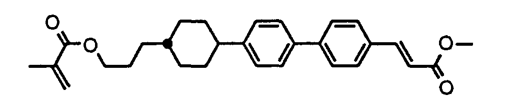

Zu einer Lösung von 6,4 ml Phosphonoessigsäuretrimethylester in 50 ml trockenem Tetrahydrofuran wurden bei 0 °C innerhalb von 10 Minuten 27,6 ml einer 1,6 n Butyllithiumlösung getropft. Es wurde 1,5 Stunden bei 0 °C gerührt und danach innerhalb von 5 Minuten bei derselben Temperatur eine Lösung von 11,5 g rohem trans-4'-[4-[3-(2-Methyl-acryloyloxy)-propyl]-cyclohexyl]-biphenyl-4-carboxaldehyd in 50 ml trockenem Tetrahydrofuran zugetropft. Anschliessend wurde langsam auf Raumtemperatur erwärmt und während 15 Stunden reagieren gelassen. Das Reaktionsgemisch wurde dann zwischen Methylenchlorid und In Salzsäure verteilt, die organische Phase mit gesättigter Natriumbicarbonatlösung und Wasser gewaschen, über Magnesiumsulfat getrocknet und eingedampft. Chromatographie an Kieselgel mit Essigester / Hexan (1:9) und anschliessende mehrfache Umkristallisation aus Hexan / Essigester ergab 0,71 g trans-2-Methyl-acrylsäure 3-[4-[4'-[(E)-2-methoxycarbonyl-vinyl]-biphenyl-4-yl]-cyclohexyl]-propyl ester als farblose Kristalle.

### Beispiel 7;

### Poly [1-[6-[4-[4-[(E)-2-methoxycarbonyl-vinyl]-phenoxycarbonyl]-phenoxy]-hexyloxycarbonyl]-1-methyl-ethylen-co-1-[2-ethylhexyloxycarbonyl]-1-methyl-ethylen]

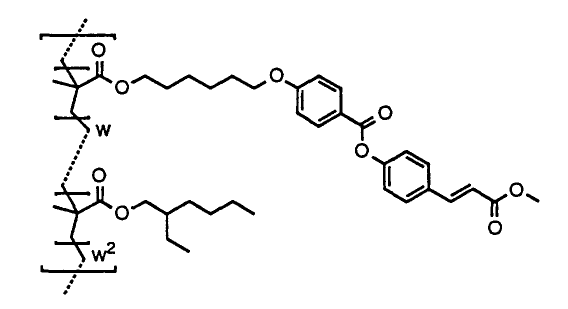

0,43 g (0,91 mmol) 4-[6-(2-Methyl-acryloyloxy)-hexyloxy]-benzoesäure 4-[(E)-2-methoxycarbonyl-vinyl]-phenyl ester, 0,01 g (0,05 mmol) Methacrylsäure-2-ethylhexylester (Fluka 64072) und 1,6 mg (0,0095 mmol) 2,2'-Azo-bis-isobutyronitril wurden in 1,9 ml Tetrahydrofuran (THF) gelöst. Die Lösung wurde 30 Minuten mit einem schwachen Argonstrom durchspült. Anschliessend wurde das Reaktionsgefäss luftdicht verschlossen und auf 55 °C erhitzt. Nach 15 Stunden wurde das Gefäss geöffnet, die Lösung mit 2 ml THF verdünnt und unter starkem Rühren bei Raumtemperatur in 0,91 Diethylether getropft. Das ausgefallene Polymer wurde abfiltriert und bei 50 °C im Wasserstrahlvakuum getrocknet. Zur weiteren Reinigung wurde das Polymer in ca. 5 ml Dichlormethan gelöst und erneut in 0,9 1 Diethylether gefällt. Dieser Vorgang wurde solange wiederholt, bis dünnschichtchromatographisch kein Monomer mehr nachweisbar war. Filtrieren und Trocknen bei 50 °C im Wasserstrahlvakuum ergaben 0,385 g Poly [1-[6-[4-[4-[(E)-2-methoxycarbonyl-vinyl]-phenoxycarbonyl]-phenoxy]-hexyloxycarbonyl]-1-methyl-ethylen-co-1-[2-ethylhexyloxycarbonyl]-1-methyl-ethylen] (18:1) mit der Phasenfolge (°C): G 42 S 205 I.

Der als Ausgangsmaterial verwendete 4-[6-(2-Methyl-acryloyloxy)-hexyloxy]-benzoesäure 4-[(E)-2-methoxycarbonyl-vinyl]-phenyl ester wurde analog zum 4-[6-(2-Methyl-acryloyloxy)-hexyloxy]-benzoesäure 2-methoxy-4-[(E)-2-methoxycarbonyl-vinyl]-phenyl ester (Beispiel 4) hergestellt.

In analoger Weise lassen sich folgende Polymere synthetisieren:
Poly [1-[6-[4-[4-[(E)-2-methoxycarbonyl-vinyl]-phenoxycarbonyl]-phenoxy]-hexyloxycarbonyl]-1-methyl-ethylen-co-1-[2-ethylhexyloxycarbonyl]-1-methyl-ethylen] (9:1), Phasenfolge (°C): G 41 LC 200 I;
Poly [1-[6-[4-[4-[(E)-2-methoxycarbonyl-vinyl]-phenoxycarbonyl]-phenoxy]-hexyloxycarbonyl]-1-methyl-ethylen-co-1-[2-ethylhexyloxycarbonyl]-1-methyl-ethylen] (4:1);
Poly [1-[3-[4-[4-[(E)-2-methoxycarbonyl-vinyl]-phenoxycarbonyl]-phenoxy]-propoxycarbonyl]-1-methyl-ethylen-co-1-[2-ethylhexyloxycarbonyl]-ethylen];
Poly [1-[2-[4-[4-[(E)-2-methoxycarbonyl-vinyl)-phenoxycarbonyl)-phenoxy]-ethoxycarbonyl]-1-methyl-ethylen-co-1-hexyloxycarbonyl-ethylen];
Poly [1-[6-[4-[4-[(E)-2-methoxycarbonyl-vinyl]-phenoxycarbonyl]phenoxy]-hexyloxycarbonyl]-1-methyl-ethylen-co-1-methoxycarbonyl-1-methyl-ethylen];
Poly [1-[6-[4-[4-[(E)-2-methoxycarbonyl-vinyl]-phenoxycarbonyl]-phenoxy]-hexyloxycarbonyl]-1-methyl-ethylen-co-1-ethoxycarbonyl-1-methylethylen];
Poly [1-[6-[4-[4-[(E)-2-methoxycarbonyl-vinyl]-phenoxycarbonyl]-phenoxy]-hexyloxycarbonyl]-1-methyl-ethylen-co-1-propoxycarbonyl-1-methylethylen];
Poly [1-[6-[4-[4-[(E)-2-methoxycarbonyl-vinyl]-phenoxycarbonyl]-phenoxy]-hexyloxycarbonyl]-1-methyl-ethylen-co-1-butyloxycarbonyl-1-methylethylen];
Poly [1-[6-[4-[4-[(E)-2-methoxycarbonyl-vinyl]-phenoxycarbonyl]-phenoxy]-hexyloxycarbonyl]-1-methyl-ethylen-co-1-pentyloxycarbonyl-1-methyl-ethylen];
Poly [1-[6-[4-[4-[(E)-2-methoxycarbonyl-vinyl]-phenoxycarbonyl]-phenoxy]-hexyloxycarbonyl]-1-methyl-ethylen-co-1-hexyloxycarbonyl-1-methylethylen];
Poly [1-[6-[4-[4-[(E)-2-methoxycarbonyl-vinyl]-phenoxycarbonyl]-phenoxy]-hexyloxycarbonyl]-1-methyl-ethylen-co-1-octyloxycarbonyl-1-methylethylen];
Poly [1-[6-[4-[4-[(E)-2-methoxycarbonyl-vinyl]-phenoxycarbonyl]-phenoxy]-hexyloxycarbonyl]-1-methyl-ethylen-co-1-dodecyloxycarbonyl-1-methyl-ethylen];
Poly [1-[6-[4-[4-[(E)-2-methoxycarbonyl-vinyl]-phenoxycarbonyl]-phenoxy]-hexyloxycarbonyl]-1-methyl-ethylen-co-1-octadecyloxycarbonyl-1-methyl-ethylen];
Poly [1-[6-[4-[4-[(E)-2-methoxycarbonyl-vinyl]-phenoxycarbonyl]phenoxy]-hexyloxycarbonyl]-1-methyl-ethylen-co-1-allyloxycarbonyl-1-methyl-ethylen];
Poly [1-[6-[4-[4-[(E)-2-methoxycarbonyl-vinyl]-phenoxycarbonyl]-phenoxy]-hexyloxycarbonyl]-1-methyl-ethylen-co-1-phenyl-ethylen];
Poly [1-[6-[4-[4-[(E)-2-methoxycarbonyl-vinyl]-phenoxycarbonyl]-phenoxy]-hexyloxycarbonyl]-1-methyl-ethylen-co-1-methoxycarbonyl-ethylen];
Poly [1-[6-[4-[4-[(E)-2-methoxycarbonyl-vinyl]-phenoxycarbonyl]-phenoxy]-hexyloxycarbonyl]-1-methyl-ethylen-co-1-butyloxycarbonyl-ethylen];
Poly [1-[6-[4-[4-[(E)-2-methoxycarbonyl-vinyl]-phenoxycarbonyl]-phenoxy]-hexyloxycarbonyl]-1-methyl-ethylen-co-1-cyano-ethylen];
Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-methoxycarbonyl-1-methyl-ethylen];
Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-ethoxycarbonyl-1-methyl-ethylen] (19:1), Tg = 76 °C;
Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-propoxycarbonyl-1-methyl-ethylen];
Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-[2-methyl-propoxycarbonyl]-1-methyl-ethylen];
Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-butyloxycarbonyl-1-methyl-ethylen];
Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-pentyloxycarbonyl-1-methyl-ethylen];
Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-hexyloxycarbonyl-1-methyl-ethylen];
Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-[2-ethylhexyloxycarbonyl-1-methyl-ethylen] (20:1), Tg = 73 °C;
Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-octyloxycarbonyl-1-methyl-ethylen];
Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-dodecyloxycarbonyl-1-methyl-ethylen];
Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-octadecyloxycarbonyl-1-methyl-ethylen];
Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-allyloxycarbonyl-1-methyl-ethylen];
Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-[2-phenoxyethoxycarbonyl]-1-methyl-ethylen];
Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-[2-phenylethoxycarbonyl]-1-methyl-ethylen];
Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-phenylethylen];
Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-cyanoethylen];
Poly [1-[4-[(E)-2-methoxycarbonyl-vinyl]-phenoxycarbonyl]-1-methylethylen-co-1-phenyl-ethylen];
Poly [1-[4-[(E)-2-methoxycarbonyl-vinyl]-phenoxycarbonyl]-1-methylethylen-co-1-methoxycarbonyl-1-methyl-ethylen];
Poly [1-[4-[(E)-2-methoxycarbonyl-vinyl]-phenoxycarbonyl]-1-methylethylen-co-1-propoxycarbonyl-1-methyl-ethylen];
Poly [1-[4-[(E)-2-methoxycarbonyl-vinyl]-phenoxycarbonyl]-1-methylethylen-co-1-hexyloxycarbonyl-1-methyl-ethylen];
Poly [1-[2-[4-[(E)-2-methoxycarbonyl-vinyl]-phenoxy]-ethoxycarbonyl]-1-methyl-ethylen-co-1-methoxycarbonyl-1-methyl-ethylen];
Poly [1-[2-[4-[(E)-2-methoxycarbonyl-Ninyl]-phenoxy]-ethoxycarbonyl]-1-methyl-ethylen-co-1-butyloxycarbonyl-1-methyl-ethylen];
Poly [1-[2-[4-[(E)-2-methoxycarbonyl-vinyl]-phenoxy]-ethoxycarbonyl]-1-methyl-ethylen-co-1-octyloxycarbonyl-1-methyl-ethylen];
Poly [1-[4-[4-[(E)-2-methoxycarbonyl-vinyl]-phenoxy]-butyloxycarbonyl]-1-methyl-ethylen-co-1-methoxycarbonyl-1-methyl-ethylen];
Poly [1-[6-[4-[(E)-2-methoxycarbonyl-vinyl]-phenoxy]-hexyloxycarbonyl]-1-methyl-ethylen-co-1-hexyloxycarbonyl-1-methyl-ethylen];
Poly [1-[2-[2-methoxy-4-[(E)-2-methoxycarbonyl-vinyl]-phenoxy]-ethoxycarbonyl]-1-methyl-ethylen-co-1-butyloxycarbonyl-1-methyl-ethylen];
Poly [1-[4-[2-methoxy-4-[(E)-2-methoxycarbonyl-vinyl]-phenoxy]-butyloxycarbonyl]-1-methyl-ethylen-co-1-pentyloxycarbonyl-1-methyl-ethylen].

### Beispiel 8:

### Poly [1-[2-[(E)-2-methoxycarbonyl-vinyl]-naphthalin-6-yloxycarbonyl]-1-methyl-ethylen]

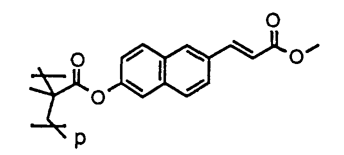

0,9 g (3,04 mmol) 2-Methyl-acrylsäure (E)-2-(2-methoxycarbonyl-vinyl)-naphthalin-6-yl ester und 3,7 mg (0,02 mmol) 2,2'-Azo-bis-isobutyronitril wurden in 4,5 ml Tetrahydrofuran (THF) gelöst. Die Lösung wurde 30 Minuten mit einem schwachen Argonstrom durchspült. Anschliessend wurde das Reaktionsgefäss luftdicht verschlossen und auf 60 °C erhitzt. Nach 9 Stunden wurde das Gefäss geöffnet, die Lösung mit 2 ml THF verdünnt und unter starkem Rühren bei Raumtemperatur in 11 Methanol getropft. Das ausgefallene Polymer wurde abfiltriert und bei 40 °C im Vakuum getrocknet. Zur weiteren Reinigung wurde das Polymer in ca. 6 ml THF gelöst und erneut in 1 1 Methanol gefällt. Dieser Vorgang wurde solange wiederholt, bis dünnschichtchromatographisch kein Monomer mehr nachweisbar war. Filtrieren und Trocknen bei 40 °C im Vakuum ergaben 0,15 g Poly [1-[2-[(E)-2-methoxycarbonyl-vinyl]-naphthalin-6-yloxycarbonyl]-1-methyl-ethylen] mit einem Molekulargewicht M_{w} von 624 000 (Gelpermeationschromatographie: THF, 35 °C, Polystyrolstandard) und Absorptionsmaxima bei λ_{max.}(CHCl₃) = 261,9 nm; 270,9 nm und 303,7 nm.

Der als Ausgangsmaterial verwendete 2-Methyl-acrylsäure (E)-2-(2-methoxycarbonyl-vinyl)-naphthalin-6-yl ester wurde nach folgendem Verfahren hergestellt.

### (E)-3-(6-Hydroxy-naphthalin-2-yl)-acrylsäure methyl ester

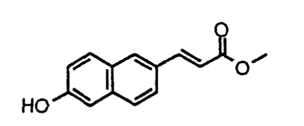

In einem mit Stickstoff begasten Kolben wurden 5 g (22,4 mmol) 6-Brom-2-naphthol, 6 ml (67,2 mmol) Methylacrylat, 100 mg (0,45 mmol) Palladiumacetat und 25 ml Triethylamin vorgelegt. Nach Zugabe von 545 mg (1,79 mmol) Tri-(o-tolyl)-phosphin wurde der Reaktionsansatz über Nacht unter Rückfluss gekocht. Nach 15 Stunden wurde die Reaktion abgebrochen und zwischen Ethylacetat und Wasser verteilt. Die organische Phase wurde dreimal mit Wasser und die wässrigen Phasen einzeln je zweimal mit Ethylacetat nachgewaschen. Die vereinigten organischen Phasen wurden über Magnesiumsulfat getrocknet, über Celit filtriert und im Vakuum vollständig eingedampft. Chromatographie an Kieselgel mit Toluol/Ethylacetat (3:2) und anschliessende Umkristallisation aus Ethylacetat ergaben 3,65 g des (E)-3-(6-Hydroxy-naphthalin-2-yl)-acrylsäure methyl esters.

### 2-Methyl-acrylsäure (E)-2-(2-methoxycarbonyl-vinyl)-naphthalin-6-yl ester

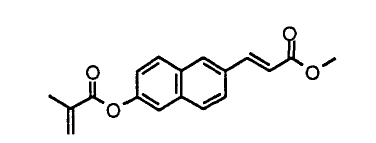

0,89 g (3,9 mmol) (E)-3-(6-Hydroxy-naphthalin-2-yl)-acrylsäure methyl ester, eine Spatelspitze BHT und 0,66 ml Triethylamin wurden in 10 ml THF gelöst. Nach Abkühlung auf 0 °C wurde innerhalb von 30 Minuten eine Lösung aus 0,39 ml (4,07 mmol) Methacrylsäurechlorid in 4 ml THF zugetropft. Die weisse Suspension wurde noch eine Stunde bei 0 °C gerührt und dann auf Ether und Wasser verteilt. Die organische Phase wurde noch zweimal mit Wasser und die wässrigen Phasen zweimal mit Diethylether nachgewaschen. Die vereinigten organischen Phasen wurden über Magnesiumsulfat getrocknet, über Celit filtriert und im Vakuum vollständig eingedampft. Das Rohprodukt wurde aus einem Gemisch aus 5 ml Hexan und 20 ml Toluol umkristallisiert. Dies ergab 0,92 g des weissen 2-Methyl-acrylsäure (E)-2-(2-methoxycarbonyl-vinyl)-naphthalin-6-yl esters.

### Beispiel 9:

### Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-[2-[4-[2-methoxy-4-[(E)-2-methoxycarbonyl-vinyl]-phenoxycarbonyl]-phenoxy]-ethoxycarbonyl]-1-methyl-ethylen]

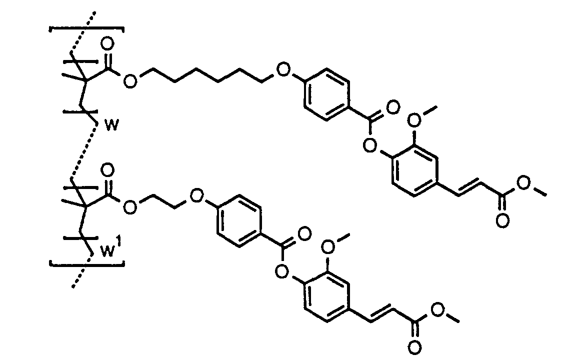

0,224 g (0,45 mmol) 4-[6-(2-Methyl-acryloyloxy)-hexyloxy]-benzoesäure 2-methoxy-4-[(E)-2-methoxycarbonyl-vinyl]-phenyl ester, 0,198 g (0,45 mmol) 4-[2-(2-Methyl-acryloyloxy)-ethoxy]-benzoesäure 2-methoxy-4-[(E)-2-methoxycarbonyl-vinyl]-phenyl ester und 1,5 mg (0,009 mmol) 2,2'-Azo-bis-isobutyronitril wurden in 1,8 ml Tetrahydrofuran (THF) gelöst. Die Lösung wurde 30 Minuten mit einem schwachen Argonstrom durchspült. Anschliessend wurde das Reaktionsgefäss luftdicht verschlossen und auf 55 °C erhitzt. Nach 6 Stunden wurde das Gefäss geöffnet, die Lösung mit 2 ml THF verdünnt und unter starkem Rühren bei Raumtemperatur in 0,9l Diethylether getropft. Das ausgefallene Polymer wurde abfiltriert und bei 40 °C im Wasserstrahlvakuum getrocknet. Zur weiteren Reinigung wurde das Polymer in ca. 5 ml Dichlormethan gelöst und erneut in 0,9 1 Diethylether gefällt. Dieser Vorgang wurde solange wiederholt, bis dünnschichtchromatographisch kein Monomer mehr nachweisbar war. Filtrieren und Trocknen bei 40 °C im Wasserstrahlvakuum ergaben 0,26 g Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-[2-[4-[2-methoxy-4-[(E)-2-methoxycarbonyl-vinyl]-phenoxycarbonyl]-phenoxy]-ethoxycarbonyl]-1-methyl-ethylen] (1:1) mit einer Glastufe bei Tg = 92 °C und einem Absorptionsmaximum von λ_{max.} (in CH₂Cl₂) = 276,5 nm .

Der 4-[6-(2-Methyl-acryloyloxy)-hexyloxy]-benzoesäure 2-methoxy-4-[(E)-2-methoxycarbonyl-vinyl]-phenyl ester und der 4-[2-(2-Methyl-acryloyloxy)-ethoxy]-benzoesäure 2-methoxy-4-[(E)-2-methoxycarbonyl-vinyl]-phenyl ester wurden nach dem in Beispiel 4 beschrieben Verfahren hergestellt.

In analoger Weise lassen sich folgende Polymere synthetisieren:
Poly [1-[3-[4-[2-methoxy-4-[(E)-2-methoxycarbonyl-vinyl]-phenoxycarbonyl]-phenoxy]-propoxycarbonyl]-1-methyl-ethylen-co-1-[2-[4-[2-methoxy-4-[(E)-2-methoxycarbonyl-vinyl]-phenoxycarbonyl]-phenoxy]-ethoxycarbonyl]-1-methyl-ethylen];
Poly [1-[3-[4-[2-methoxy-4-[(E)-2-methoxycarbonyl-vinyl]-phenoxycarbonyl]-phenoxy]-propoxycarbonyl]-1-methyl-ethylen-co-1-[6-[4-[2-methoxy-4-[(E)-2-methoxycarbonyl-vinyl]-phenoxycarbonyl]-phenoxy]-hexyloxycarbonyl]- 1-methyl-ethylen];
Poly [1-[2-[4-[2-methoxy-4-[(E)-2-methoxycarbonyl-Ninyl]-phenoxycarbonyl]-phenoxy]-ethoxycarbonyl]-1-methyl-ethylen-co-1-[8-[4-[2-methoxy-4-[(E)-2-methoxycarbonyl-vinyl]-phenoxycarbonyl]-phenoxy]-octyloxycarbonyl]- 1-methyl-ethylen];
Poly [1-[6-[4-[2-ethoxy-4-[(E)-2-methoxycarbonyl-vinyl]-phenoxycarbonyl]-phenoxy]-hexyloxycarbonyl]-1-methyl-ethylen-co-1-[2-[4-[2-methoxy-4-[(E)-2-methoxycarbonyl-vinyl]-phenoxycarbonyl]-phenoxy]-ethoxycarbonyl]-1-methylethylen];
Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-[2-[4-[4-[(E)-2-methoxycarbonyl-vinyl]-phenoxycarbonyl]-phenoxy]-ethoxycarbonyl]-1-methyl-ethylen];
Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-[2-[4-[2-fluoro-4-[(E)-2-methoxycarbonyl-vinyl]-phenoxycarbonyl]-phenoxy]-ethoxycarbonyl]-1-methyl-ethylen];
Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-[2-[4-[2-methoxy-4-[(E)-2-propoxycarbonyl-vinyl]-phenoxycarbonyl]-phenoxy]-ethoxycarbonyl]- 1-methyl-ethylen];
Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-[2-[4-[2-methoxy-4-[(E)-2-hexyloxycarbonyl-vinyl]-phenoxycarbonyl]-phenoxy]-ethoxycarbonyl]-1-methyl-ethylen];
Poly [1-[6-[2-methoxy-4-[4-[(E)-2-methoxycarbonyl-vinyl]-phenoxycarbonyl]-phenoxy]-hexyloxycarbonyl]-1-methyl-ethylen-co-1-[2-[4-[2-methoxy-4-[(E)-2-methoxycarbonyl-vinyl]-phenoxycarbonyl]-phenoxy]-ethoxycarbonyl]-1-methyl-ethylen];
Poly [1-[6-[2-methoxy-4-[4-[(E)-2-methoxycarbonyl-vinyl]-phenoxycarbonyl]-phenoxy]-hexyloxycarbonyl]-1-methyl-ethylen-co-1-[2-[4-[4-[(E)-2-butyloxycarbonyl-vinyl]-phenoxycarbonyl]-phenoxy]-ethoxycarbonyl]-1-methyl-ethylen];
Poly [1-[6-[2-methoxy-4-[3-fluoro-4-[(E)-2-methoxycarbonyl-vinyl]-phenoxycarbonyl]-phenoxy]-hexyloxycarbonyl]-1-methyl-ethylen-co-1-[2-[4-[2-methoxy-4-[(E)-2-methoxycarbonyl-vinyl]-phenoxycarbonyl]-phenoxy]-ethoxycarbonyl]-1-methyl-ethylen];
Poly [1-[6-[2-methoxy-4-[4-[(E)-2-methoxycarbonyl-vinyl]-phenoxycarbonyl]-phenoxy]-hexyloxycarbonyl]-1-methyl-ethylen-co-1-[2-[4-[(E)-2-methoxycarbonyl-vinyl]-phenoxy]-ethoxycarbonyl]-1-methyl-ethylen];
Poly [1-[6-[4-[2-fluoro-4-[(E)-2-methoxycarbonyl-vinyl]-phenoxycarbonyl]-phenoxy]-hexyloxycarbonyl]-1-methyl-ethylen-co-1-[6-[4-[4-[(E)-2-methoxycarbonyl-vinyl]-phenyl]-phenoxy]-hexyloxycarbonyl]-1-methyl-ethylen];
Poly [1-[8-[4-[2-methoxy-4-[(E)-2-methoxycarbonyl-vinyl]-phenoxycarbonyl]-phenoxy]-octyloxycarbonyl]-1-methyl-ethylen-co-1-[3-[4-[4'-[(E)-2-methoxycarbonyl-vinyl]-biphenyl-4-yl]-cyclohexyl]-propoxycarbonyl]-1-methyl-ethylen];
Poly[1-[6-[4-[2-methoxy-4-[(E)-2-methoxycarbonyl-vinyl]-phenoxycarbonyl]-phenoxy]-hexyloxycarbonyl]-1-methyl-ethylen-co-1-[6-[2-[(E)-2-methoxycarbonyl-vinyl]-naphthalin-6-yl]-hexyloxycarbonyl]-1-methylethylen].

## Patentansprüche

1. Copolymerzusammensetzungen mit wiederkehrenden Einheiten der allgemeinen Formel Ia, 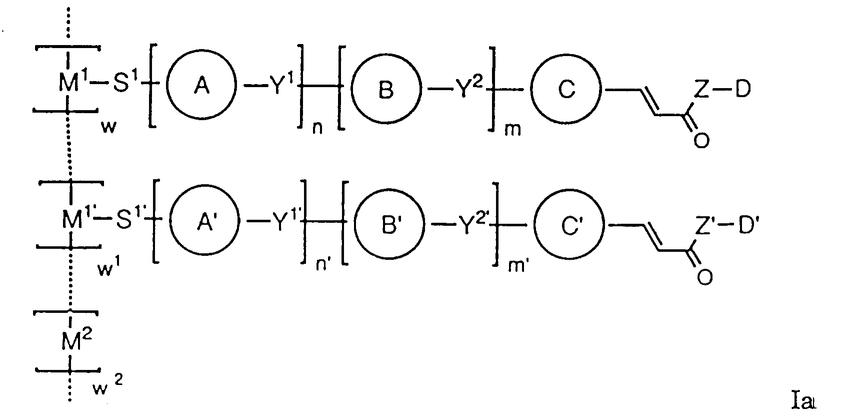 und einem Molekulargewicht zwischen 1'000 und 5'000'000, worin
M¹ eine wiederkehrende Monomereinheit aus der Gruppe Acrylat, Methacrylat, 2-Chloracrylat, 2-Phenylacrylat; gegebenenfalls durch niederes Alkyl N-substituiertes Acrylamid, Methacrylamid, 2-Chloracrylamid und 2-Phenylacrylamid; Vinylether, Vinylester, Styrol-Derivate, Siloxane;
S¹ Spacereinheiten, wie beispielsweise eine einfache kovalente Bindung, eine gegebenenfalls einfach oder mehrfach mit Fluor-, Chlor- oder Cyano-substituierte geradkettige oder verzweigte Alkylengruppierung im Folgenden repräsentiert durch -(CH₂)ᵣ-, sowie -(CH₂)ᵣ-O-, -(CH₂)ᵣ-O-(CH₂)ₛ-, -(CH₂)ᵣ-O-(CH₂)ₛ-O-, -(CH₂)ᵣ-CO-, -(CH₂)ᵣ-CO-O-, -(CH₂)ᵣ-O-CO-, -(CH₂)ᵣ-NR²-, -(CH₂)ᵣ-CO-NR²-, -(CH₂)ᵣ-NR²-CO-, -(CH₂)ᵣ-NR²-CO-O- oder -(CH₂)ᵣ-NR²-CO-NR³-, wobei r und s jeweils eine ganze Zahl von 1 bis 20 ist, mit der Massgabe, dass r + s ≤ 20, und R² und R³ unabhängig voneinander Wasserstoff oder niederes Alkyl;
Ring A unsubstituiertes oder gegebenenfalls mit Fluor, Chlor, Cyano, Alkyl oder Alkoxy substituiertes Phenylen, Pyridin-2,5-diyl, Pyrimidin-2,5-diyl, 1,3-Dioxan-2,5-diyl, Cyclohexan-1,4-diyl, Piperidin-1,4-diyl, Piperazin-1,4-diyl;
Ring B unsubstituiertes oder gegebenenfalls mit Fluor, Chlor, Cyano, Alkyl oder Alkoxy substituiertes Phenylen, Pyridin-2,5-diyl, Pyrimidin-2,5-diyl, 1,4- bzw. 2,6-Naphthylen, 1,3-Dioxan-2,5-diyl, Cyclohexan-1,4-diyl;
Y¹, Y² unabhängig voneinander eine einfache Kovalenzbindung, -(CH₂)ₜ-, -O-, -CO-, -CO-O-, -O-OC-, -NR⁴-, -CO-NR⁴-, -R⁴N-CO-, -(CH₂)ᵤ-O-, -O-(CH₂)ᵤ-, -(CH₂)ᵤ-NR⁴- oder -NR⁴-(CH₂)ᵤ-, worin
Ring C unsubstituiertes oder gegebenenfalls mit Fluor, Chlor, Cyano, Alkyl oder Alkoxy substituiertes Phenylen, oder Pyrimidin-2,5-diyl, Pyridin-2,5-diyl, 2,5-Thiophenylen, 2,5-Furanylen, 1,4- oder 2,6-Naphthylen;
Z -O- oder -NR⁵-, wobei R⁵ Wasserstoff oder niederes Alkyl, oder eine zweite Gruppe der Formel D, wobei
D eine gegebenenfalls mit Fluor oder Chlor substituierte geradkettige oder verzweigte Alkylgruppe mit 1 bis 20 Kohlenstoffatomen, ein gegebenenfalls mit Fluor, Chlor, Alkyl oder Alkoxy substituierter Cycloalkylrest mit 3 bis 8 Ringatomen bedeuten; M^{1'}, S^{1'}, A', B', C', D', Z', Y^{1'}, Y^{2'}, m' und n' die unter M¹, S¹, A, B, C, D, Z, Y¹, Y², m und n angegebene Bedeutung haben; und
M² eine wiederkehrende Monomereinheit aus der Gruppe: Acrylat, Methacrylat, 2-Chloracrylat, 2-Phenylacrylat, gegebenenfalls durch niederes Alkyl N-substituiertes Acrylamid, Methacrylamid, 2-Chloracrylamid und 2-Phenylacrylamid; Vinylether, Vinylester; geradkettige oder verzweigte Alkylester der Acryl- bzw. Methacrylsäure, Allylester der Acryl- bzw. Methacrylsäure, Alkylvinylether bzw. Ester, Phenoxyalkylacrylate bzw. Phenoxyalkylmethacrylate oder Hydroxyalkylacrylate bzw. Hydroxyalkylmethacrylate, Phenylalkylacrylate bzw. Phenylalkylmethacrylate, wobei die Alkylreste 1 bis 20 Kohlenstoffatome haben; Acrylnitril, Methacrylnitril, Styrol, 4-Methylstyrol oder Siloxane bedeutet;
w, w¹ und w² Molenbrüche der Comonomeren mit 0< w <1, 0 <w¹<1 und 0 < w² ≤ 0,5 sind.

2. Copolymerzusammensetzungen nach Anspruch 1 mit wiederkehrenden Einheiten der allgemeinen Formel Ia, worin
Ring A unsubstituiertes oder gegebenenfalls mit Fluor, Chlor, Cyano, Alkyl oder Alkoxy substituiertes Phenylen, Pyridin-2,5-diyl, Pyrimidin-2,5-diyl oder Cyclohexan-1,4-diyl;
Ring B unsubstituiertes oder gegebenenfalls mit Fluor, Chlor, Cyano, Alkyl oder Alkoxy substituiertes Phenylen, Pyridin-2,5-diyl, Pyrimidin-2,5-diyl, 1,4- bzw. 2,6-Naphthylen oder Cyclohexan-1,4-diyl;
Y¹, Y² unabhängig voneinander eine einfache Kovalenzbindung, -CH₂CH₂-, -O-, -CH₂-O-, -O-CH₂-, -CO-O- oder -O-OC-;
m, n unabhängig voneinander 0 oder 1;
Ring C unsubstituiertes oder gegebenenfalls mit Fluor, Chlor, Cyano, Alkyl oder Alkoxy substituiertes Phenylen, oder Pyrimidin-2,5-diyl, Pyridin-2,5-diyl, 2,5-Furanylen oder 1,4- oder 2,6-Naphthylen;
Z -O-; und
D eine geradkettige oder verzweigte Alkylgruppe mit 1 bis 20 Kohlenstoffatomen oder ein gegebenenfalls mit Alkyl oder Alkoxy substituierter Cycloalkylrest mit 5 oder 6 Ringatomen;
bedeuten;
A', B', C', D', Z', Y^{1'}, Y^{2'}, m' und n' die unter A, B, C, D, Z, Y¹, Y², m und n angegebene Bedeutung haben; und
w, w¹ und w² Molenbrüche der Comonomeren mit 0< w <1, 0 <w¹<1 und 0<w²≤ 0,5 sind.

3. Copolymerzusammensetzungen nach Anspruch 2, worin n und n' = 0 ist.

4. Copolymerzusammensetzungen nach Anspruch 3, worin
Ring B unsubstituiertes oder gegebenenfalls mit Fluor, Chlor, Cyano, Alkyl oder Alkoxy substituiertes Phenylen, Pyridin-2,5-diyl, Pyrimidin-2,5-diyl oder Cyclohexan-1,4-diyl;
Y² eine einfache Kovalenzbindung, -CO-O- oder -O-OC-;
m 0 oder 1;
Ring C unsubstituiertes oder gegebenenfalls mit Fluor, Chlor, Cyano, Alkyl oder Alkoxy substituiertes Phenylen oder 1,4- oder 2,6-Naphthylen;
n 0;
Z -O-; und
D eine geradkettige oder verzweigte Alkylgruppe mit 1 bis 12 Kohlenstoffatomen;
bedeuten;
B', C', D', Z', Y^{2'} und m' die unter B, C, D, Z, Y² und m angegebene Bedeutung haben; und
w, w¹ und w² Molenbrüche der Comonomeren mit 0< w <1, 0 <w¹<1 und 0<w ²≤0,5 sind.

5. Copolymerzusammensetzung nach Anspruch 4,
Poly [1-[2-[4-[(E)-2-methoxycarbonyl-vinyl]-phenoxy]-ethoxycarbonyl]-1-methyl-ethylen-co-1-[2-[4-[(E)-2-propoxycarbonyl-vinyl]-phenoxy]-ethoxycarbonyl]-1-methyl-ethylen-co-1-[2-hydroxv-ethoxycarbonyl]-1-methyl-ethylen].

6. Copolymerzusammensetzungen mit wiederkehrenden Einheiten der allgemeinen Formel Ib, 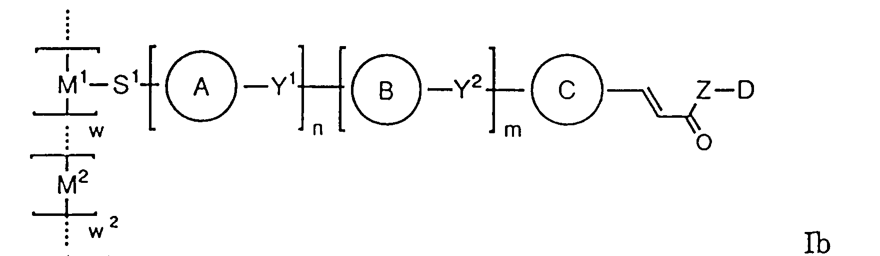 und einem Molekulargewicht zwischen 1'000 und 5'000'000 worin
M¹, S¹, A, B, C, D, Z, Y¹, Y², m und n die in Anspruch 1 angegebene Bedeutung haben;
M² die in Anspruch 1 angegebene Bedeutung hat;
w und w ² Molenbrüche der Comonomeren mit 0 < w < 1 und 0 < w²≤0,5 sind.

7. Copolymerzusammensetzungen nach Anspruch 6 mit wiederkehrenden Einheiten der allgemeinen Formel I b, worin
A, B, C, D, Z, Y¹, Y², m und n die in Anspruch 2 angegebene Bedeutung haben;
w und w 2 Molenbrüche der Comonomeren mit 0 < w < 1 und 0 < w ²≤ 0,5 sind.

8. Copolymerzusammensetzungen nach Anspruch 7, worin n = 0 ist.

9. Copolymerzusammensetzungen nach Anspruch 8, worin
B, C, D, Z, Y² und m die in Anspruch 4 angegebene Bedeutung haben;
w und w 2 Molenbrüche der Comonomeren mit 0 < w < 1 und 0 < w ²≤0,5 sind.

10. Copolymerzusammensetzungen nach Anspruch 9,
Poly [1-[6-[4-[4-[(E)-2-methoxycarbonyl-vinyl]-phenoxycarbonyl]-phenoxy]-hexyloxycarbonyl]-1-methyl-ethylen-co-1-[2-ethylhexyloxycarbonyl]-1-methyl-ethylen];
Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-ethoxycarbonyl-1-methyl-ethylen];
Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-[2-ethylhexyloxycarbonyl-1-methyl-ethylen].

11. Copolymerzusammensetzung mit wiederkehrenden Einheiten der allgemeinen Formel Ic, 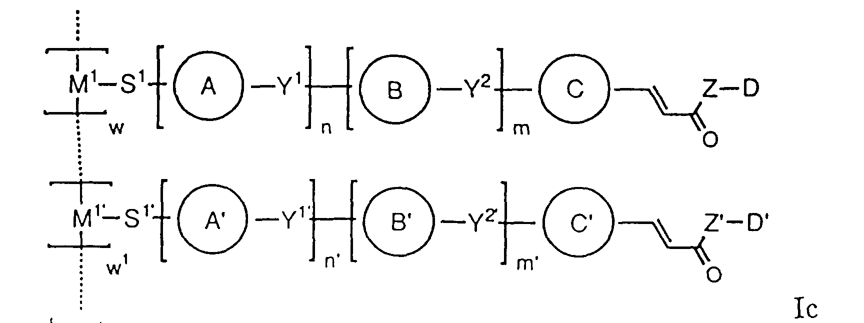 und einem Molekulargewicht zwischen 1'000 und 5'000'000.
worin
M¹, S¹, A, B, C, D, Z, Y¹, Y², m und n die in Anspruch 1 angegebene Bedeutung haben;
M^{1'}, S^{1'}, A', B', C', D', Z', Y^{1'}, Y^{2'}, m' und n' die in Anspruch 1 unter M¹, S¹, A, B, C, D, Z, Y¹, Y², m und n angegebene Bedeutung haben;
w und w¹ Molenbrüche der Comonomeren mit 0< w <1 und 0 <w¹<1 sind.

12. Copolymerzusammensetzung nach Anspruch 11 mit wiederkehrenden Einheiten der allgemeinen Formel Ic, worin
M¹ und S¹ die in Anspruch 1 angegebene Bedeutung haben;
M^{1'} und S^{1'} die in Anspruch 1 unter M¹, S¹ angegebene Bedeutung haben;
A, B, C, D, Z, Y¹, Y², m und n die in Anspruch 2 angegebene Bedeutung haben;
A', B', C', D', Z', Y^{1'}, Y^{2'}, m' und n' die in Anspruch 2 unter A, B, C, D, Z, Y¹, Y², m und n angegebene Bedeutung haben;
w und w¹ Molenbrüche der Comonomeren mit 0< w <1 und 0 <w¹<1 sind.

13. Copolymerzusammensetzung nach Anspruch 12, worin n und n' = 0 ist.

14. Copolymerzusammensetzung nach Anspruch 13, worin
M¹ und S¹ die in Anspruch 1 angegebene Bedeutung haben;
M^{1'} und S^{1'} die in Anspruch 1 unter M¹, S¹ angegebene Bedeutung haben;
B, C, D, Z, Y² und m die in Anspruch 4 angegebene Bedeutung haben;
B', C', D', Z', Y^{2'} und m' die in Anspruch 4 unter B, C, D, Z, Y² und m angegebene Bedeutung haben;
w und w¹ Molenbrüche der Comonomeren mit 0< w <1 und 0 <w¹<1 sind.

15. Copolymerzusammensetzung nach Anspruch 14,
Poly [1-[6-[4-[2-methoxy-4-[(E)-2-methoxycarbonyl-vinyl]-phenoxycarbonyl]-phenoxy]-hexyloxycarbonyl]-1-methyl-ethylen-co-1-[2-[4-[2-methoxy-4-[(E)-2-methoxycarbonyl-vinyl]-phenoxycarbonyl]-phenoxy]-ethoxycarbonyl]-1-methyl-ethylen].

16. Homopolymerzusammensetzungen mit wiederkehrenden Einheiten der allgemeinen Formel I, 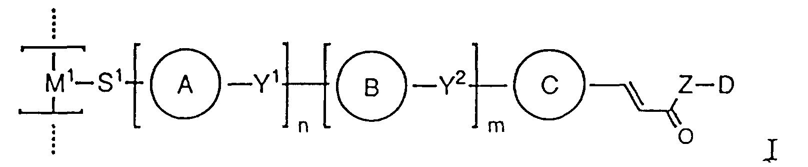 und einem Molekulargewicht zwischen 1'000 und 5'000'000
worin
M¹, S¹, A, B, C, D, Z, Y¹, Y² m und n die in Anspruch 1 angegebene Bedeutung haben.

17. Homopolymerzusammensetzungen nach Anspruch 16,
Poly [1-[3-[4-[4'-[(E)-2-methoxycarbonyl-vinyl]-biphenyl-4-yl]-cyclohexyl]-propoxycarbonyl]-1-methyl-ethylen].

18. Homopolymerzusammensetzungen nach Anspruch 16 mit wiederkehrenden Einheiten der allgemeinen Formel I, worin
M¹ und S¹ die in Anspruch 1 angegebene Bedeutung haben;
A, B, C, D, Z, Y¹, Y² m und n die in Anspruch 2 angegebene Bedeutung haben.

19. Homopolymerzusammensetzungen nach Anspruch 18, worin n = 0 ist.

20. Homopolymerzusammensetzungen nach Anspruch 19, worin
M¹ und S¹ die in Anspruch 1 angegebene Bedeutung haben;
B, C, D, Z, Y² und m die in Anspruch 4 angegebene Bedeutung haben.

21. Homopolymerzusammensetzungen nach Anspruch 20,
Poly [1-[4-[(E)-2-methoxycarbonyl-vinyl]-phenoxycarbonyl]-1-methylethylen];
Poly [1-[4-[(E)-2-methoxycarbonyl-vinyl]-phenylaminocarbonyl]-1-methylethylen];
Poly [1-[2-[4-[(E)-2-methoxycarbonyl-vinyl]-phenoxy]-ethoxycarbonyl]-1-methyl-ethylen];
Poly [1-[6-[4-[2-methoxy-4-[(E)-2-methoxycarbonyl-vinyl]-phenoxycarbonyl]-phenoxy]-hexyloxycarbonyl]-1-methyl-ethylen];
Poly [1-[6-[4-[4-[(E)-2-methoxycarbonyl-vinyl]-phenoxycarbonyl]-phenoxy]-hexyloxycarbonyl]-1-methyl-ethylen];
Poly [oxy-[4-[4-[4-[(E)-2-methoxycarbonyl-vinyl]-phenoxycarbonyl]-phenoxy]-butyl]-methyl-silylen];
Poly [1-[2-[(E)-2-methoxycarbonyl-vinyl]-naphthalin-6-yloxycarbonyl]-1-methyl-ethylen].

22. Elektro-optische Vorrichtungen, **dadurch gekennzeichnet, dass** photoaktive Polymermaterialien mit 3-Arylacrylsäure-estem und -amiden nach einem der Ansprüche 1 bis 21, als Orientierungsschichten für Flüssigkristalle verwendet werden.

23. Verwendung von vernetzbaren, photoaktiven Polymermaterialien mit 3-Aryl-acrylsäureestern und -amiden nach einem der Ansprüche 1 bis 21, zum Aufbau unstrukturierter bzw. strukturierter optischer Elemente und Mehrschichtsysteme.

## Claims

1. Copolymer compositions with repeating units of general formula Ia, 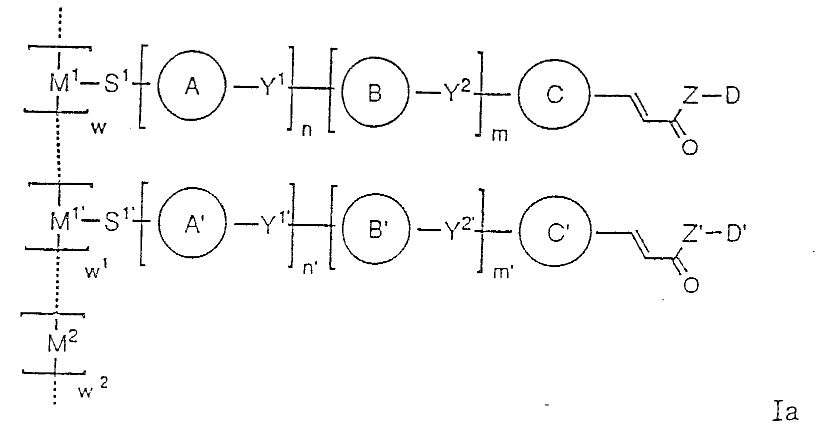 and a molecular weight of 1,000 to 5,000,000 wherein
M¹ signifies a repeating monomer unit from the group acrylate, methacrylate, 2-chloroacrylate, 2-phenylacrylate; unsubstituted or N-lower alkyl substituted acrylamide, methacrylamide, 2-chloroacrylamide and 2-phenylacrylamide; vinyl ether, vinyl ester, styrene derivative, siloxane;
S¹ signifies spacer units such as, for example, a single covalent bond, a straight-chain or branched alkylene grouping represented hereinafter by -(CH₂)ᵣ-, as well as -(CH₂)ᵣ-O-, -(CH₂)ᵣ-O-(CH₂)ₛ-, - (CH₂)ᵣ-O-(CH₂)_{S}-O-- (CH₂)ᵣ-CO-, -(CH₂)ᵣ-CO-O-, -(CH₂)ᵣ-O-CO-, -(CH₂)ᵣ-NR²-, -(CH₂)ᵣ-CO-NR²-, -(CH₂)ᵣ-NR²-CO-, -(CH₂)ᵣ-NR²-CO-O- or -(CH₂)ᵣ-NR²-CO-NR³- which is optionally mono- or multiply substituted with fluorine, chlorine or cyano and in which r and s are each a whole number of 1 to 20, with the proviso that r + s ≤ 20, and R² and R³ each independently signify hydrogen or lower alkyl;
ring A signifies phenylene, pyridine-2,5-diyl, pyrimidine-2,5-diyl, 1,3-dioxane-2,5-diyl, cyclohexane-1,4-diyl, piperdine-1,4-diyl, piperazine-1,4-diyl which is unsubstituted or optionally substituted with fluorine, chlorine, cyano, alkyl or alkoxy;
ring B signifies phenylene, pyridine-2,5-diyl, pyrimidine-2,5-diyl, 1,4- or 2,6-naphthylene, 1,3-dioxane-2,5-diyl, cyclohexane-1,4-diyl which is unsubstituted or optionally substituted with fluorine, chlorine, cyano, alkyl or alkoxy;
Y¹, Y² each independently signify a single covalent bond -(CH₂)ₜ-, -O-, -CO-, -CO-O-, -O-OC-, -NR⁴-, -CO-NR⁴-, -R⁴N-CO-, -(CH₂)ᵤ-O-, -O-(CH₂)ᵤ-, -(CH₂)ᵤ-NR⁴- or -NR⁴-(CH₂)ᵤ-, in which
ring C signifies phenylene which is unsubstituted or optionally substituted with fluorine, chlorine, cyano, alkyl or alkoxy, or pyrimidine-2,5-diyl, pyridine-2,5-diyl, 2,5-thiophenylene, 2,5-furanylene, 1,4- or 2,6-naphthylene;
Z signifies -O- or - NR⁵-, in which R⁵ signifies hydrogen or lower alkyl, or a second group of formula D, in which
D signifies a straight-chain or branched alkyl group with 1 to 20 carbon atoms which is optionally substituted with fluorine or chlorine, a cycloalkyl residue with 3 to 8 ring atoms which is optionally substituted with fluorine, chlorine, alkyl or alkoxy;
M¹, S¹, A', B', C', D', Z', Y^{1'}, Y^{2'}, m' and n' are as defined under M¹, S¹, A, B, C, D, Z, Y¹, Y², m and n; and
M² signifies a repeating monomer unit from the group; acrylate, methacrylate, 2-chloroacrylate, 2-phenylacrylate; unsubstituted or N-lower alkyl substituted acrylamide, methacrylamide, 2-chloroacrylamide and 2-phenylacrylamide; vinyl ether, vinyl ester, straight-chain or branched alkyl esters of acrylic or methacrylic acid, allyl esters of acrylic or methacrylic acid, alkyl vinyl ethers or esters, phenoxyalkyl acrylates or phenoxyalkyl methacrylates, or hydroxyalkyl acrylates or hydroxyalkyl methacrylates phenylalkyl acrylates or phenylalkyl methacrylates, with alkyl residues of 1 to 20 carbon atoms; acrylonitrile, methacrylonitrile, styrene, 4-methylstrene, siloxane;
w, w¹ and w² are molar fractions of the comonomers with 0 < w < 1, 0 < w¹ < 1 and 0 < w² ≤ 0,5.

2. Copolymer compositions according to claim 1 with repeating units of the general formula Ia, wherein
ring A signifies phenylene, pyridine-2,5-diyl, pyrimidine-2,5-diyl or cyclohexane-1,4-diyl which is unsubstituted or optionally substituted with fluorine, chlorine, cyano, alkyl or alkoxy;
ring B signifies phenylene, pyridine-2,5-diyl, pyrimidine-2,5-diyl, 1,4- or 2,6-naphthylene or cyclohexane-1,4-diyl which is unsubstituted or optionally substituted with fluorine, chlorine, cyano, alkyl or alkoxy;
Y¹, Y² each independently signify a single covalent bond - CH₂CH₂-, -O-, -CH₂-O-, -O-CH₂-, -CO-O- or -O-OC;
m, n each independently signify 0 or 1;
ring C signifies phenylene which is unsubstituted or optionally substituted with fluorine, chlorine, cyano, alkyl or alkoxy, or pyrimidine-2,5-diyl, pyridine-2,5-diyl, 2,5-furanylene, 1,4- or 2,6-naphthylene;
Z signifies -O-, and
D signifies a straight-chain or branched alkyl group with 1 to 20 carbon atoms or a cycloalkyl residue with 5 to 6 ring atoms which is optionally substituted with alkyl or alkoxy;
A', B', C', D', Z', Y^{1'}, Y^{2'}, m' and n' are as defined under A, B, C, D, Z, Y¹, Y², m and n; and
w, w¹ and w² are molar fractions of the comonomers with 0 < w < 1, 0 < w¹ < 1 and 0 < w² ≤ 0,5.

3. Copolymer compositions according to claim 2, wherein n and n' signify 0.

4. Copolymer compositions according to claim 3, wherein
ring B signifies phenylene, pyridine-2,5-diyl, pyrimidine-2,5-diyl or cyclohexane-1,4-diyl which is unsubstituted or optionally substituted with fluorine, chlorine, cyano, alkyl or alkoxy;
Y² signifies a single covalent bond, -CO-O- or -O-OC-;
m signifies 0 or 1;
ring C signifies phenylene which is unsubstituted or optionally substituted with fluorine, chlorine, cyano, alkyl or alkoxy or 1,4- or 2,6-naphthylene;
n signifies 0;
Z signifies -O- and
D signifies a straight-chain or branched alkyl group with 1 to 12 carbon atoms;
B', C', D', Z', Y^{2'} and m' are as defined under B, C, D, Z, Y² and m; and
w, w¹ and w² are molar fractions of the comonomers with 0 < w < 1, 0 < w¹ < 1 and 0 < w² ≤ 0,5.

5. Copolymer compositions according to claim 4,
poly [1-[2-[4-[(E)-2-methoxycarbonyl-vinyl]-phenoxy]-ethoxycarbonyl]-1-methyl-ethylene-co-1-[2-[4-[(E)-2-propoxycarbonyl-vinyl]-phenoxy]-ethoxycarbonyl]-1-methylethylene-co-1-[2-hydroxy-ethoxycarbonyl]-1-methylethylene].

6. Copolymer compositions with repeating units of the general formula Ib 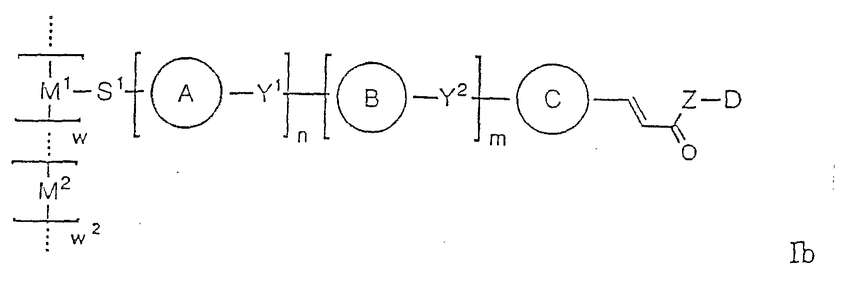 and a molecular weight of 1,000 to 5,000,000 wherein
M¹, S¹, A, B, C, D, Z, Y¹, Y², m and n are as defined in claim 1;
M² is as defined in claim 1;
w and w² are molar fractions of the comonomers with 0 < w < 1 and 0 < w² ≤ 0,5.

7. Copolymer compositions according to claim 6 with repeating units of the general formula Ib, wherein
A, B, C, D, Z, Y¹, Y², m and n are as defined in claim 2; w and w² are molar fractions of the comonomers with 0 < w < 1 and 0 < w² ≤ 0,5.

8. Copolymer compositions according to claim 7, wherein n signifies 0.

9. Copolymer compositions according to claim 8, wherein
B, C, D, Z, Y² and m are as defined in claim 4;
w and w² are molar fractions of the comonomers with 0 < w < 1 and 0 < w² ≤ 0,5.

10. Copolymer compositions according to claim 9
poly [1-[6-[4-[4-[(E)-2-methoxycarbonyl-vinyl]-phenoxycarbonyl]-phenoxy]-hexyloxycarbonyl]-1-methylethylene-co-1-[2-ethylhexyloxycarbonyl]-1-methylethylene];
poly [1-[6-[4-[2-methoxy-4-[(E)-2-methoxycarbonyl-vinyl]-phenoxycarbonyl]-phenoxy]-hexyloxycarbonyl]-1-methylethylene-co-1-ethoxycarbonyl-1-methyl-ethylene];
poly [1-[6-[4-[2-methoxy-4- (E)-2-methoxycarbonyl-vinyl]-phenoxycarbonyl]-phenoxy]-hexyloxycarbonyl]-1-methylethylene-co-1-[2-ethylhexyloxycarbonyl-1-methylethylene].

11. Copolymer compositions with repeating units of the general formula Ic, 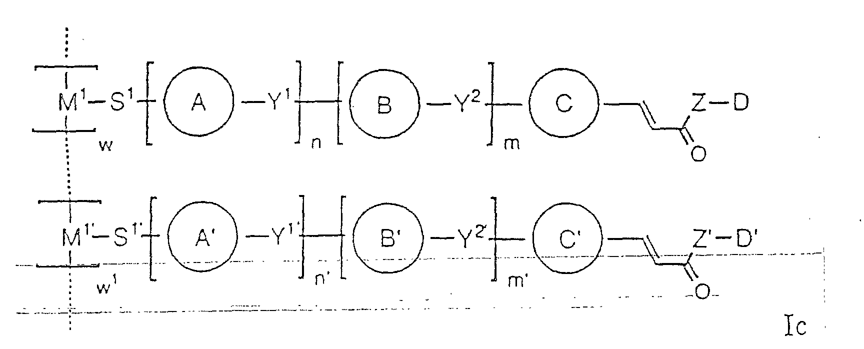 and a molecular weight of 1,000 to 5,000,000 wherein
M¹, S¹, A, B, C, D, Z, Y¹, Y², m and n are as defined in claim 1;
M^{1'}, S^{1'}, A', B', C', D', Z', Y^{1'}, Y^{2'}, m' and n' are as defined in claim 1 under M¹, S¹, A, B, C, D, Z, Y¹, Y², m and n;
w and w¹ are molar fractions of the comonomers with 0 < w < 1 and 0 < w¹ < 1.

12. Copolymer compositions according to claim 11, with repeating units of the general formula Ic, wherein
M¹ and S¹ are as defined in claim 1,
M^{1'} and S^{1'} are as defined in claim 1 under M¹, S¹;
A, B, C, D, Z, Y¹, Y², m and n are as defined in claim 2;
A', B', C', D', Z', Y^{1'}, Y^{2'}, m' and n' are as defined in claim 2 under A, B, C, D, Z, Y¹, Y², m and n;
w and w¹ are molar fractions of the comonomers with 0 < w < 1 and 0 < w¹ < 1.

13. Copolymer compositions according to claim 12, wherein n and n' signify 0.

14. Copolymer compositions according to claim 13, wherein
M¹ and S¹ are as defined in claim 1;
M^{1'} and S^{1'} are as defined in claim 1 under M¹, S¹;
B, C, D, Z, Y² and m are as defined in claim 4;
B', C', D', Z', Y^{2'} and m' are as defined in claim 4 under B, C, D, Z, Y² and m;
w and w¹ are molar fractions of the comonomers with 0 < w < 1 and 0 < w¹ < 1.

15. Copolymer compositions according to claim 14,
poly [1-[6-[4-[2-methoxy-4-[(E)-2-methoxycarbonyl-vinyl]-phenoxycarbonyl]-phenoxy]-hexyloxycarbonyl]-1-methylethylene-co-1-[2-[4-[2-methoxy-4-[(E)-2-methoxycarbonyl]-vinyl]-phenoxycarbonyl]-phenoxy]-ethoxycarbonyl]-1-methyl-ethylene].

16. Homopolymer compositions with repeating units of the general formula I 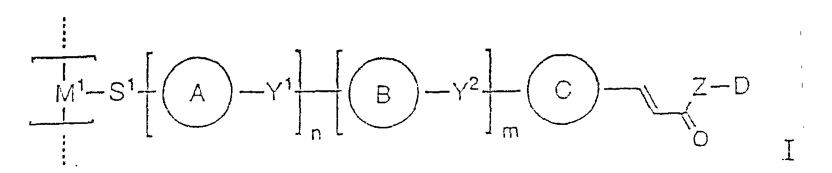 and a molecular weight of 1,000 to 5,000,000 wherein
M¹, S¹, A, B, C, D, Z, Y¹, Y², m and n are as defined in claim 1.

17. Homopolymer compositions according to claim 16,
poly [1-[3-[4-[4'-[(E)-2-methoxycarbonyl-vinyl]-biphenyl-4-yl]-cyclohexyl]-propoxycarbonyl]-1-methyl-ethylene].

18. Homopolymer compositions according to claim 16, with repeating units of the general formula I, wherein
M¹ and S¹ are as defined in claim 1;
A, B, C, D, Z, Y¹, Y², m and n are as defined in claim 2.

19. Homopolymer compositions according to claim 18, wherein n signifies 0.

20. Homopolymer compositions according to claim 19, wherein
M¹ and S¹ are as defined in claim 1;
B, C, D, Z, Y² and m are as defined in claim 4.

21. Homopolymer compositions according to claim 20
poly[1-[4-[(E)-2-methoxycarbonyl-vinyl]-phenoxycarbonyl]-1-methyl-ethylene];
poly[1-[4-[(E)-2-methoxycarbonyl-vinyl]-phenylaminocarbonyl]-1-methyl-ethylene];
poly[1-[2-[4-[(E)-2-methoxycarbonyl-vinyl]-phenoxy]-ethoxycarbonyl]-1-methyl-ethylene];
poly[1-[6-[4-[2-methoxy-4-[(E)-2-methoxycarbonyl-vinyl]-phenoxycarbonyl]-phenoxy]-hexyloxycarbonyl]-1-methylethylene];
poly[1-[6-[4-[4-[(E)-2-methoxycarbonyl-vinyl]-phenoxycarbonyl]-phenoxy]-hexyloxycarbonyl]-1-methylethylene];
poly[oxy-[4-[4-[4-[(E)-2-methoxycarbonyl-vinyl]-phenoxycarbonyl]-phenoxy]-butyl]-methyl-silylen];
poly[1-[2-[(E)-2-methoxycarbonyl-vinyl]-naphthalin-6-yloxycarbonyl]-1-methyl-ethylene].

22. Electro-optical devices, **characterised in that** photoactive polymer materials with 3-aryl-acrylic acid esters and amides according to any one of claims 1 to 21 are used as orienting layers for liquid crystals.

23. Use of crosslinkable, photoactive polymer materials with 3-aryl-acrylic acid esters and amides according to any one of claims 1 to 21 for the production of non-structured or structured optical elements and multi-layer systems.

## Revendications

1. Compositions de copolymères ayant des motifs répétés de formule générale Ia, 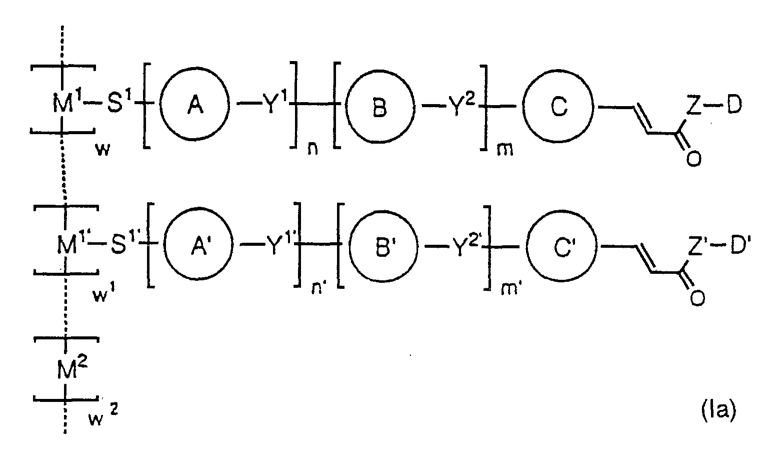 et une masse moléculaire comprise entre 1000 et 5 000 000, dans laquelle
M¹ représente un motif monomère répété choisi parmi le groupe acrylate, méthacrylate, 2-chloroacrylate, 2-phénylacrylate ; acrylamide, méthacrylamide, 2-chloroacrylamide et 2-phénylacrylamide éventuellement substitué sur l'azote par un groupe alkyle inférieur ; vinyléther, vinylester, dérivés de styrène, siloxanes ;
S¹ représente des motifs espaceurs, comme par exemple une liaison covalente simple, un groupement alkylène à chaîne linéaire ou ramifiée, éventuellement substitué une ou plusieurs fois avec un fluor, un chlore ou un cyano, représenté dans ce qui suit par -(CH₂)ᵣ-, ainsi que -(CH₂)ᵣ-O-, -(CH₂)ᵣ-O-(CH₂)ₛ-, -(CH₂)ᵣ-O-(CH₂)ₛ-O-, -(CH₂)ᵣ-CO-, -(CH₂)ᵣ-CO-O-, -(CH₂)ᵣ-O-CO-, -(CH₂)ᵣ-NR²-, -(CH₂)ᵣ-CO-NR²-, -(CH₂)ᵣ-NR²-CO-, -(CH₂)ᵣ-NR²-CO-O-ou -(CH₂)ᵣ-NR²-CO-NR³-, dans lesquels r et s représentent respectivement un nombre entier de 1 à 20, à la condition que r + s ≤ 20, et R² et R³ sont indépendamment l'un de l'autre un hydrogène ou un alkyle inférieur ;
le cycle A représente un groupe phénylène, pyridine-2,5-diyle, pyrimidine-2,5-diyle, 1,3-dioxane-2,5-diyle, cyclohexane-1,4-diyle, pipéridine-1,4-diyle, pipérazine-1,4-diyle, non substitué ou éventuellement substitués par du fluor, chlore, cyano, alkyle ou alcoxy ;
le cycle B représente un groupe phénylène, pyridine-2,5-diyle, pyrimidine-2,5-diyle, 1,4-ou 2,6-naphtylène, 1,3-dioxane-2,5-diyle, cyclohexane-1,4-diyle, non substitués ou éventuellement substitué par du fluor, chlore, un cyano, alkyle ou alcoxy ;
Y¹, Y² représentent indépendamment l'un de l'autre une liaison simple covalente, -(CH₂)ₜ-, -O-, -CO-, -CO-O-, -O-OC-, -NR⁴-, -CO-NR⁴-, -R⁴N-CO-, -(CH₂)ᵤ-O-, -O-(CH₂)ᵤ-, -(CH₂)ᵤ-NR⁴- ou -NR⁴-(CH₂)ᵤ-, dans lesquels
le cycle C représente un groupe phénylène, ou pyrimidine-2,5-diyle, pyridine-2,5-diyle, 2,5-thiophénylène, 2,5-furanylène, 1,4- ou 2,6-naphtylène, non substitué ou éventuellement substitué par du fluor, chlore, cyano, alkyle ou alcoxy ;
Z représente -O- ou -NR⁵, dans lequel R⁵ représente un hydrogène ou un groupe alkyle inférieur, ou un deuxième groupe de formule D, dans laquelle
D représente un groupe alkyle éventuellement substitué avec du fluor ou du chlore, à chaîne linéaire ou ramifiée, ayant de 1 à 20 atomes de carbone, un groupe cycloalkyle éventuellement substitué par du fluor, chlore, alkyle ou alcoxy, ayant de 3 à 8 atomes de cycle ;
M¹', S¹', A', B', C', D', Z', Y^{1'}, Y^{2'}, m' et n' ont la signification indiquée pour M¹, S¹, A, B, C, D, Z, Y¹, Y², m et n ; et
M² représente un motif monomère répété choisi parmi le groupe acrylate, méthacrylate, 2-chloroacrylate, 2-phénylacrylate, acrylamide, méthacrylamide, 2-chloroacrylamide et 2-phénylacrylamide éventuellement N-substitué par un groupe alkyle inférieur ; vinyléther, vinylester ; un alkylester à chaîne linéaire ou ramifiée de l'acide acrylique ou méthacrylique, allylester de l'acide acrylique ou méthacrylique, alkylvinyléther ou ester, acrylate de phénoxyalkyle ou méthacrylate de phénoxyalkyle, acrylate d'hydroxyalkyle ou méthacrylate d'hydroxyalkyle, acrylate de phénylalkyle ou méthacrylate de phénylalkyle, dans lesquels les groupes alkyle ont de 1 à 20 atomes de carbone ; acrylonitrile, méthacrylonitrile, styrène, 4-méthyl-styrène ou siloxanes ; w, w¹ et w² sont les proportions molaires des comonomères avec 0 < w < 1, 0 < w¹ < 1 et 0 < w² ≤ 0,5.

2. Compositions de copolymères selon la revendication 1 avec les motifs répétés de formule générale Ia, dans laquelle
le cycle A représente un groupe phénylène, pyridine-2,5-diyle, pyrimidine-2,5-diyle ou cyclohexane-1,4-diyle, non substitué ou éventuellement substitué avec du fluor, chlore, cyano, alkyle ou alcoxy ;
le cycle B représente un groupe phénylène, pyridine-2,5-diyle, pyrimidine-2,5-diyle, 1,4-ou 2,6-naphtylène ou cyclohexane-1,4-diyle, non substitué ou éventuellement substitué avec du fluor, chlore, cyano, alkyle ou alcoxy ;
Y¹, Y² représentent indépendamment l'un de l'autre une liaison de covalence simple, -CH₂CH₂-, -O-, -CH₂-O-, -O-CH₂-, -CO-O- ou -O-OC- ;
m et n sont indépendamment l'un de l'autre 0 ou 1 ;
le cycle C représente un groupe phénylène, ou pyrimidine-2,5-diyle, pyridine-2,5-diyle, 2,5-furanylène ou 1,4- ou 2,6-naphtylène, non substitué ou éventuellement substitué avec du fluor, chlore, cyano, alkyle ou alcoxy ;
Z représente -O- ; et
D représente un groupe alkyle à chaîne linéaire ou ramifiée ayant de 1 à 20 atomes de carbone ou un groupe cycloalkyle ayant 5 ou 6 atomes de cycle éventuellement substitué avec un alkyle ou un alcoxy ;
A', B', C', D', Z', Y^{1'}, Y^{2'}, m' et n' ont la signification indiquée pour A, B, C, D, Z, Y¹, Y², m et n ; et
w, w¹ et w² sont les proportions molaires des comonomères avec 0 < w < 1, 0 < w¹ < 1 et 0 < w² ≤ 0,5.

3. Compositions de copolymères selon la revendication 2, dans lesquelles n et n' sont égaux à 0.

4. Compositions de copolymères selon la revendication 3, dans lesquelles
le cycle B représente un groupe phénylène, pyridine-2,5-diyle, pyrimidine-2,5-diyle ou cyclohexane-1,4-diyle, non substitué ou éventuellement substitué par du fluor, chlore, cyano, alkyle ou alcoxy ;
Y² représente une liaison de covalence simple, -CO-O- ou -O-OC- ;
m est 0 ou 1 ;
le cycle C représente un groupe phénylène, ou 1,4- ou 2,6-napthylène non substitué ou éventuellement substitué avec du fluor, chlore, cyano, alkyle ou alcoxy ;
n est 0 ;
Z représente -O- ; et
D représente un groupe alkyle à chaîne linéaire ou ramifiée ayant de 1 à 12 atomes de carbone ;
B', C', D', Z', Y^{2'} et m' ont la signification indiquée pour B, C, D, Z, Y² et m ; et w, w¹ et w² sont les proportions molaires des comonomères avec 0 < w < 1, 0 < w¹ < 1 et 0 < w² ≤ 0,5.

5. Composition de copolymères selon la revendication 4,
poly[1-[2-[4-[(E)-2-méthoxycarbonyl-vinyl]-phénoxy]-éthoxycarbonyl]-1-méthyl-éthylène-co-1-[2-[4-[(E)-2-propoxycarbonyl-vinyl]-phénoxy]-éthoxy-carbonyl]-1-méthyl-éthylène-co-1-[2-hydroxy-éthoxycarbonyl]-1-méthyl-éthylène].

6. Compositions de copolymères avec des motifs répétés de formule générale Ib, 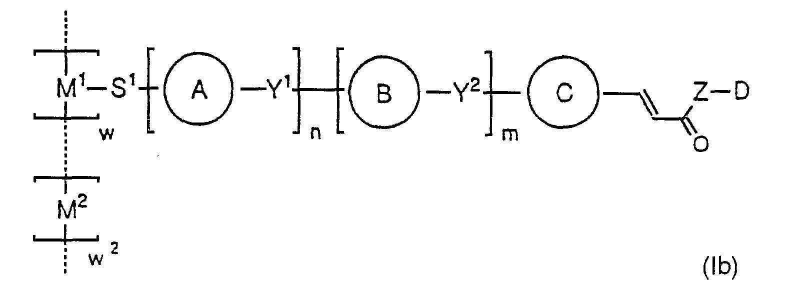 et une masse moléculaire comprise entre 1000 et 5 000 000, dans laquelle
M¹, S¹, A, B, C, D, Z, Y¹, Y², m et n ont la signification indiquée à la revendication 1 ;
M² a la signification indiquée à la revendication 1 ;
w et w² sont les proportions molaires des comonomères avec 0 < w < 1 et 0 < w² ≤ 0,5.

7. Compositions de copolymères selon la revendication 6 avec des motifs répétés de formule générale Ib, dans laquelle
A, B, C, D, Z, Y¹, Y², m et n ont la signification indiquée à la revendication 2 ; w et w² sont les proportions molaires des comonomères avec 0 < w < 1 et 0 < w² ≤ 0,5.

8. Compositions de copolymères selon la revendication 7, dans lesquelles n est égal à 0.

9. Compositions de copolymères selon la revendication 8, dans lesquelles B, C, D, Z, Y² et m ont la signification indiquée à la revendication 4 ; w et w² sont les proportions molaires des comonomères avec 0 < w < 1 et 0 < w² ≤ 0,5.

10. Compositions de copolymères selon la revendication 9,
poly[1-[6-[4-[4-[(E)-2-méthoxycarbonyl-vinyl]-phénoxycarbonyl]-phénoxy]-hexyloxycarbonyl]-1-méthyl-éthylène-co-1-[2-éthylhexyloxycarbonyl]-1-méthyléthylène] ;
poly[1-[6-[4-[2-méthoxy-4- [(E)-2-méthoxycarbonyl-vinyl]-phénoxycarbonyl]-phénoxy]-hexyloxycarbonyl]-1-méthyl-éthylène-co-1-éthoxycarbonyl-1-méthyléthylène] ;
poly[1-[6-[4-[2-méthoxy-4-[(E)-2-méthoxycarbonyl-vinyl]-phénoxycarbonyl]-phénoxy]-hexyloxycarbonyl]-1-méthyl-éthylène-co-1-[2-éthylhexyloxycarbonyl-1-méthyl-éthylène].

11. Composition de copolymères avec des motifs répétés de formule générale Ic, 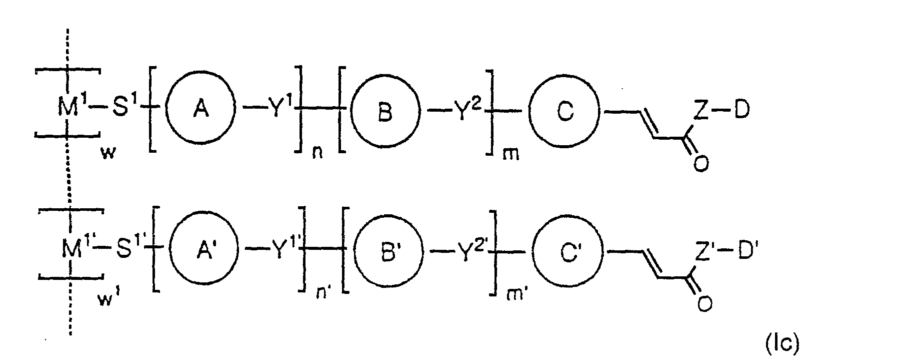 et une masse moléculaire comprise entre 1000 et 5 000 000, dans laquelle
M¹, S¹, A, B, C, D, Z, Y¹, Y², m et n ont la signification indiquée à la revendication 1 ;
M¹', S¹', A', B', C', D', Z', Y^{1'}, Y²', m' et n' ont la signification indiquée à la revendication 1 pour M¹, S¹, A, B, C, D, Z, Y¹, Y², m et n ;
w et w¹ sont les proportions molaires des comonomères avec 0 < w < 1 et 0 < w¹ < 1.

12. Composition de copolymères selon la revendication 11 avec des motifs répétés de formule générale Ic, dans laquelle
M¹ et S¹ ont la signification indiquée à la revendication 1 ;
M^{1'} et S^{1'} ont la signification indiquée à la revendication 1 pour M¹, S¹ ;
A, B, C, D, Z, Y¹, Y², m et n ont la signification indiquée à la revendication 2 ;
A', B', C', D', Z', Y^{1'}, Y^{2'}, m' et n' ont la signification indiquée à la revendication 2 pour A, B, C, D, Z, Y¹, Y², m et n ;
w¹ et w² sont les proportions molaires des comonomères avec 0 < w < 1 et 0 < w¹ < 1.

13. Composition de copolymères selon la revendication 12, dans laquelle n et n' sont égaux à 0.

14. Composition de copolymères selon la revendication 13, dans laquelle
M¹ et S¹ ont la signification indiquée à la revendication 1 ;
M^{1'} et S^{1'} ont la signification indiquée à la revendication 1 pour M¹, S¹ ;
B, C, D, Z, Y² et m ont la signification indiquée à la revendication 4 ;
B', C', D', Z', Y^{2'} et m' ont la signification indiquée à la revendication 4 pour B, C, D, Z, Y² et m ;
w et w¹ sont les proportions molaires des comonomères avec 0 < w < 1 et 0 < w¹ < 1.

15. Composition de copolymères selon la revendication 14,
poly[1-[6-[4-[2-méthoxy-4-[(E)-2-méthoxycarbonyl-vinyl]-phénoxycarbonyl]-phénoxy]-hexyloxycarbonyl]-1-méthyl-éthylène-co-1-[2-[4-[2-méthoxy-4-[(E)-2-méthoxycarbonyl-vinyl]-phénoxycarbonyl]-phénoxy]-éthoxycarbonyl]-1-méthyléthylène].

16. Compositions d'homopolymères ayant des motifs répétés de formule générale I, 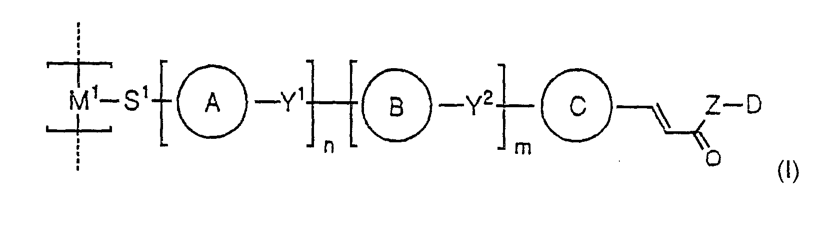 et une masse moléculaire comprise entre 1000 et 5 000 000, dans laquelle
M¹, S¹, A, B, C, D, Z, Y¹, Y², m et n ont la signification indiquée à la revendication 1.

17. Compositions d'homopolymères selon la revendication 16,
poly[1-[3-[4-[4'-[(E)-2-méthoxycarbonyl-vinyl]-biphényl-4-yl]-cyclohexyl]-propoxycarbonyl]-1-méthyl-éthylène.

18. Compositions d'homopolymères selon la revendication 16 ayant des motifs répétés de formule générale I, dans laquelle
M¹ et S¹ ont la signification indiquée à la revendication 1 ;
A, B, C, D, Z, Y¹, Y², m et n ont la signification indiquée à la revendication 2.

19. Compositions d'homopolymères selon la revendication 18, dans lesquelles n est égal à 0.

20. Compositions d'homopolymères selon la revendication 19, dans lesquelles
M¹ et S¹ ont la signification indiquée à la revendication 1 ;
B, C, D, Z, Y² et m ont la signification indiquée à la revendication 4.

21. Compositions d'homopolymères selon la revendication 20,
poly[1-[4-[(E)-2-méthoxycarbonyl-vinyl]-phénoxycarbonyl]-1-méthyléthylène] ;
poly[1-[4-[(E)-2-méthoxycarbonyl-vinyl]-phénylaminocarbonyl]-1-méthyléthylène] ;
poly[1-[2-[4-[(E)-2-méthoxycarbonyl-vinyl]-phénoxy]-éthoxycarbonyl]-1-méthyl-éthylène] ;
poly[1-[6-[4-[2-méthoxy-4-[(E)-2-méthoxycarbonyl-vinyl]-phénoxycarbonyl]-phénoxy]-hexyloxycarbonyl]-1-méthyl-éthylène];
poly[1-[6-[4-[4-[(E)-2-méthoxycarbonyl-vinyl]-phénoxycarbonyl]-phénoxy]-hexyloxycarbonyl]-1-méthyl-éthylène] ;
poly[oxy-[4-[4-[4-[(E)-2-méthoxycarbonyl-vinyl]-phénoxycarbonyl]-phénoxy]-butyl]-méthyl-silylène] ;
poly[1-[2-[(E)-2-méthoxycarbonyl-vinyl]-naphtalène-6-yloxycarbonyl]-1-méthyl-éthylène].

22. Dispositifs électro-optiques, **caractérisés en ce que** des matériaux polymères photoactifs avec des esters et amides d'acide 3-arylacrylique selon l'une des revendications 1 à 21 sont utilisés comme couches d'orientation pour des cristaux liquides.

23. Utilisation de matériaux polymères photoactifs, réticulables, avec des esters et amides d'acide 3-arylacrylique selon l'une des revendications 1 à 21, pour la constructions d'éléments optiques non structurés ou structurés et de systèmes multicouches.
